(19) Europäisches Patentamt
European Patent Office
Office européen des brevets

(11) EP 1 519 713 B1

(12) **EUROPÄISCHE PATENTSCHRIFT**

(45) Veröffentlichungstag und Bekanntmachung des Hinweises auf die Patenterteilung:
**15.09.2010 Patentblatt 2010/37**

(21) Anmeldenummer: **03763811.1**

(22) Anmeldetag: **10.07.2003**

(51) Int Cl.:
***A61K 9/00*** *(2006.01)* ***A61K 9/70*** *(2006.01)*
***A61L 31/10*** *(2006.01)* ***A61L 31/14*** *(2006.01)*
***A61L 31/16*** *(2006.01)* ***A61K 31/7036*** *(2006.01)*
***A61K 31/496*** *(2006.01)* ***A61K 31/435*** *(2006.01)*
***A61K 31/4178*** *(2006.01)*

(86) Internationale Anmeldenummer:
**PCT/EP2003/007515**

(87) Internationale Veröffentlichungsnummer:
**WO 2004/006885 (22.01.2004 Gazette 2004/04)**

(54) **WIRKSTOFFFREISETZUNGSSYSTEME AUF BASIS VON BIOABBAUBAREN ODER BIOKOMPATIBLEN POLYMEREN MIT FORMGEDAECHTNISEFFEKT**

SYSTEMS FOR RELEASING ACTIVE INGREDIENTS, BASED ON BIODEGRADABLE OR BIOCOMPATIBLE POLYMERS WITH A SHAPE MEMORY EFFECT

SYSTEMES DE LIBERATION DE PRINCIPE ACTIF A BASE DE POLYMERES BIODEGRADABLES OU BIOCOMPATIBLES A MEMOIRE DE FORME

(84) Benannte Vertragsstaaten:
**AT BE BG CH CY CZ DE DK EE ES FI FR GB GR HU IE IT LI LU MC NL PT RO SE SI SK TR**

(30) Priorität: **10.07.2002 US 395167 P**

(43) Veröffentlichungstag der Anmeldung:
**06.04.2005 Patentblatt 2005/14**

(73) Patentinhaber: **GKSS-Forschungszentrum Geesthacht GmbH**
**21502 Geesthacht (DE)**

(72) Erfinder:
- **LENDLEIN, Andreas**
  **14167 Berlin (DE)**
- **STEUER, Susi**
  **52074 Aachen (DE)**
- **TULEWEIT, Annika**
  **23552 Lübeck (DE)**

(74) Vertreter: **Gulde Hengelhaupt Ziebig & Schneider**
**Patentanwälte - Rechtsanwälte**
**Wallstrasse 58/59**
**10179 Berlin (DE)**

(56) Entgegenhaltungen:
**EP-A- 0 415 671**
**EP-A- 0 422 693**
**EP-A- 1 000 958**
**WO-A-02/41929**
**WO-A-99/42147**
**WO-A-99/42528**
**US-A- 6 160 084**
**US-A1- 2001 009 982**
**US-A1- 2003 153 972**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Wirkstofffreisetzungssysteme auf Basis von bioabbaubaren oder biokompatiblen Polymeren mit Formgedächtniseffekt, Verfahren zur Herstellung der Wirkstofffreisetzungssysteme und Polymere mit Formgedächtniseffekt, die zur Herstellung der Wirstofffreisetzungssysteme geeignet sind, wobei der Form-Gedächtnis- Effekt zur Voranderung der Wirkstoffreisezumgsrate eingesezt wird.

## Stand der Technik

Wirkstofffreisetzungssysteme, die eine kontrollierte Freisetzung eingeschlossene Wirkstoffe erst am gewünschten Ziel aus ermöglichen, sind seit langem Gegenstand der Forschung. Da die herkömmliche Gabe von Wirkstoffen mit kurzzeitig hohen und anschließend kontinuierlich abfallenden Konzentrationen des Wirkstoffs verbunden ist, treten häufig toxische Wirkstoffkonzentrationen in Kombination mit unerwünschten Wirkungen und unwirksame Konzentrationen ohne die angestrebte Wirkung auf. Dies führte zur Entwicklung von zahlreichen polymeren Freisetzungssystemen, die eine Möglichkeit darstellen, die Sicherheit und Effektivität einer Wirkstoffgabe durch kontrollierte Freisetzung über einen definierten Zeitraum zu erhöhen.

Derartige, teilweise auf dem Markt erhältliche Systeme zeigen allerdings verschiedene Nachteile. Biostabile Implantate müssen beispielsweise nach der Arzneistofffreisetzung durch eine zweite Operation wieder entfernt werden. Bekannte abbaubare Polymersysteme neigen andererseits zu unerwünschten Veränderungen der mechanischen Festigkeit während der Freisetzung, da teilweise schon bei einem sehr geringen Abbaugrad die mechanischen Eigenschaften sehr stark zurückgehen.

Konventionelle Wirkstoffreisetzungssysteme zeigen zusätzlich den Nachteil, dass sperrige Implantate nur mit relativ großem Aufwand implantiert werden können, was insbesondere auch bei Implantaten der Fall ist, die gleichzeitig als Wirkstoffreisetzungssystem agieren sollen. Hier wären Implantate von Vorteil, die eine weniger aufwendige Einführung in den Körper ermöglichen, mit oder ohne zusätzliche Beeinflussung der Wirkstoffreisetzung.

WO0241929 offenbart röhrenförmige Gefäßimplantate (Stent), die ein biokompatibles thermoplastisches Material (Polyurethan) mit Formgedächtnis und Rückstelltemperatur sowie einen, zwei oder mehrere Wirkstoffe hydrophiler, hydrophober, nieder- und hochmolekularer Natur umfassen. Die Wirkstoffe werden auf die innere und/oder äußere Oberfläche der Gefäßimplantate aufgebracht oder während des Tauchverfahrens in verschiedenen Konzentrationen in einzelnen Schichten eingetragen, um das Freisetzungsprofil zu modellieren. Die Wirkstoffe können bei der Polymerisation direkt dem Monomer oder bei der Verarbeitung der Polyurethanschmelze oder -lösung zum Gefäßimplantat in gewünschter Menge zugegeben werden. Die Wirkstoffe werden im Polymer gelöst oder dispergiert, wobei das Lösen des Wirkstoffes sowohl in der Schmelze als auch in der organischen Lösung des Polymers durchgeführt werden kann.

US6160084, WO9942528, WO9942147 beschreiben Zusammensetzungen von Wirkstofffreisetzungssystemen (Stent, Katheter, Prothesen, Implantate etc.) bioabbaubarer und biokompatibler Form-Gedächtnis-Polymere bestehend aus einem harten und weichen Segment. Eine therapeutische, diagnostisch oder prophylaktisch wirksame Komponente ist in der Zusammensetzung enthalten, deren Freisetzungsrate durch den hydrolytischen Abbau des Form-Gedächtnis-Materials bestimmt ist.

EP0415671 offenbart ein System zur gesteuerten und verzögerten Freisetzung eines Wirkstoffs im Magen. Die Zusammensetzung des Freisetzungssystems umfasst Form-Gedächtnis-Polymere und einen Arzneistoff, vorzugsweise in einer Retardformulierung, die für die verzögerte Freisetzung verantwortlich ist.

EP1000958 legt bioabbaubare und absorbierbare Form-Gedächtnis-Polymer-Zusammensetzungen offen, die als Polymerkomponente Segmente aus Caprolactonen, Dioxanon, Lactiden, Glycoliden u. a. und als Wirkstoffkomponente Heilmittel, Antibiotika, Knochenwachstumsfaktoren, Hormone, Wirkstoffe, Cytokine enthalten. Der Form-Gedächtnis-Effekt wird durch die Temperatur erzielt. Die Zusammensetzungen werden für vaskuläre Anastomosen, Sehnen-, Knochenfixierung, Nahtmaterial verwendet. Freisetzung des Wirkstoffs erfolgt über hydrolytischen Abbau des Polymers.

EP0422693 beschreibt Katheter, deren Zusammensetzung einen Arzneistoff und Polymere (Blockpolymere) umfasst, deren Formgedächtnis durch Temperatur verändert wird.

US2001/09982 offenbart einen superelastischen, beschichteten Stent, der eine Vielzahl biegsamer Stränge aus einem Form-Gedächtnis-Material (Nickel-Titan-Legierung oder Form-Gedächtnis-Polymer) in einem Rohr aus Kollagen, aus einem biokompatiblen thermoplastischen oder thermoelastischen Polymer besteht.

WO03068288 bezieht sich auf implantier- oder einsetzbare Arzneimittelfreigabesysteme, die zumindest zu einem Teil im Körper abgebaut werden können.

## Aufgabe der Erfindung

Daher hat es sich die vorliegende Erfindung zur Aufgabe gestellt, Wirkstofffreisetzungssysteme anzugeben, die die oben geschilderten Nachteile des Standes der Technik zumindest teilweise überwinden. Dabei soll mindestens

eine der folgenden beiden Ziele erreicht werden:

1. Eine Steuerung der Wirkstoffreisetzung (Release) ist on-demand möglich
2. Sperrige Implantate können minimalinvasiv eingeführt werden

Die Wirkstofffreisetzungssysteme der vorliegenden Erfindung sollen bevorzugt den eingeschlossenen Wirkstoff je nach Bedarf gleichmäßig über einen bestimmten Zeitraum oder kontrolliert nach Aussetzung gegenüber einem äußeren Reiz freisetzen.

**Kurze Beschreibung der Erfindung**

Diese Aufgabe wird erfindungsgemäß durch das Wirkstofffreisetzungssystem nach Anspruch 1 gelöst. Bevorzugte Ausführungsformen dieses Systems sind in den Unteransprüchen angegeben. Weiterhin stellt die vorliegende Erfindung ein Verfahren zur Herstellung von Wirkstofffreisetzungssystemen zur Verfügung, sowie polymere Materialien, geeignet zum Einsatz in den Wirkstofffreisetzungssystemen.

**Kurze Beschreibung der Figuren**

Figur 1 zeigt den Einfluß der Wirkstoffbeladung auf die Schmelztemperatur eines Multiblockcopolymers aus Paradioxanon- und Caprolactonsegmenten.
Figur 2 zeigt den Einfluß der Wirkstoffbeladung auf die thermischen Eigenschaften von Caprolacton-co-glycolid-Netzwerken unterschiedlicher Segmentlängen. Figur 3 zeigt das Abbauverhalten amorpher, wirkstoffhaltiger Netzwerke.
Figur 4 zeigt die Wirkstofffreisetzung aus amorphen Netzwerken.
Figur 5 zeigt die Wirkstofffreisetzung aus kristallinen Netzwerken.
Figur 6 veranschaulicht die Methode des Dip-Coatings zur Modifizierung von Wirkstofffrei-Setzungssystemen.
Figur 7 verdeutlicht den Aufbau von Layer-Systemen.
Figur 8 zeigt die Modifizierung des Release von Gentamicin (G5) durch Dip Coating.
Figur 9 zeigt die Modifizierung des Release von Gentamicin (G5) durch Herstellung von Layer-Systemen.
Figur 10 zeigt unterschiedliche schematische Darstellungen erfindungsgemäßer Wirkstifffreisetzungssysteme

Die Beladung des Multiblockcopolymers basierend auf Paradioxanon- und Caprolactoneinheiten mit Arzneistoffen unterschiedlicher Polarität und unterschiedlicher Konzentration zeigt keinen signifikanten Einfluss auf die Lage des Schmelzpunkts des Schaltsegmentes, d.h. die Temperatur des SM-Überganges wird nicht wesentlich verändert (Figur 1). Die Beladung der Polyurethan-Netzwerke basierend auf Lactid-co-Glycolidsegmenten mit Arzneistoffen unterschiedlicher Polarität in einer Konzentration von 1 wt.% zeigt ebenfalls keinen Einfluss auf die Glasübergangstemperatur, d.h. die Lage des SM-Übergangs bleibt unverändert. Ein ähnliches Ergebnis wurde für Caprolacton-co-glycolid-Netzwerke unterschiedlicher Segmentlängen gefunden, die mit Ethacridinlactat beladen wurden (Figur 2).

Wirkstofffreisetzung

Figur 3 verdeutlicht, dass der Massenverlust der Netzwerke ab einer Abbauzeit von ca. 50 Tagen einsetzt. Für die Freisetzung von Wirkstoffen bedeutet dies, dass innerhalb der ersten 50 Tage mit einer diffusionskontrollierten Freisetzung zu rechnen ist, danach geht die Wirkstofffreisetzung mit dem Abbau der Matrix einher.

Die Wirkstofffreisetzung aus der amorphen Matrix N-LG(18)-10 verläuft für Nitrofurantoin und Enoxacin stufenförmig, was für lactat- und glycolathaltige Copolymermatrices typisch ist (Figur 4). Der hydrophilere Wirkstoff Ethacridinlactat wird im Vergleich zu Nitrofurantoin und Enoxacin beschleunigt freigesetzt Zu Beginn der Freisetzung wird häufig ein burst-Effekt beobachtet, wobei an der Oberfläche haftender Wirkstoff an das Medium abgegeben wird. Das zweite Intervall ist geprägt durch die diffusionskontrollierte Freisetzung der Substanz aus dem Inneren der Matrix an die Oberfläche. Mit fortschreitender Freisetzung wird der restliche Anteil des Wirkstoffs unter rapider Polymererosion freigesetzt (vgl. Figur 3). Dieser Effekt wird in der Literatur auch als *dose dumping* bezeichnet.

Die Wirkstofffreisetzung kann durch Dip-Coating (Figur 6) oder durch die Herstellung von Layer-Systemen (Figur 7) optimiert werden.

Im Falle des Paradioxanon/ Caprolacton Multiblockcopolymers erfolgt ein Burstrelease am Anfang, ein Plateau wird innerhalb von 2 Tagen erreicht. Eine Modifizierung erfolgt mit Hilfe von Dip Coating mit demselben Polymer bzw. mit der Herstellung von Layer-Systemen, wobei ein wirkstoffhaltiger Film von beiden Seiten mit reinem Polymer abgedeckt

wird. Hierdurch wird der anfängliche Burst vermieden und im Falle der Layer-Systeme eine lineare Freisetzung über mehrere Wochen erzielt (Figur 8 und 9).

## Detaillierte Beschreibung der Erfindung

Das oben genannte Ziel der Steuerung der Wirkstoffreisetzung on-demand kann erreicht werden, wenn eine Änderung des Diffusionsverhaltens bei Anwendung eines externen Stimulus erreicht wird. Derartige Wirkstoffreisetzungssyteme lassen sich erfindungsgemäß wie folgt darstellen (siehe auch Figur 10):

1. Der Wirkstoff ist in einem Depot vorgesehen, das mit einer Membran verschlossen ist. Entweder die Membran oder das Depot (oder beide) umfassen ein SMP Material.
2. Der Wirkstoff liegt verteilt, d.h. gelöst oder anders eingekapselt, in einer Matrix aus SMP Material vor. Diese kann optional m mit einer Beschichtung umgeben sein oder verhindert als solche das Austreten des Wirkstoffs.
3. Unterschiedliche Wirkstoffe sind in verschiedenen Depots oder unterschiedlichen Bereichen der Matrix oder unterschiedlichen Matrixsystemen vorgesehen. Die jeweiligen Depots können durch unterschiedliche Membranen verschlossen sein.

Die Freisetzung kann durch die folgenden Optionen erreicht werden:

1. Bei Systemen, die den Wirkstoff in einem Depot (das bevorzugt nicht aus einem SMP Material besteht) enthalten, verschlossen durch eine Membran aus einem SMP Material, kann die Freisetzung dadurch erfolgen, dass der SM Effekt ausgelöst wird, wodurch sich der Zustand der Membran ändert, von undurchlässig für den Wirkstoff zu durchlässig (entweder durch eine Zerstörung der Membran oder durch eine Änderung der Durchlässigkeit durch Veränderung der Porenstruktur oder der Kristallinität; asymmetrische Membranen sind eine weitere Option). Bei Systemen, die mehrere Depots umfassen, mit unterschiedlichen Wirkstoffen, kann ebenfalls der EM Effekt für die Freisetzung genutzt werden. Sind die unterschiedlichen Depots durch Membranen aus verschiedenen SMP Materialien verschlossen, die nicht durch den gleichen Stimulus beeinflußt werden, so kann durch eine derartige Ausgestaltung eine zeitlich abgestufte Freisetzung erreicht werden.

2. Alternativ kann bei den Depotsystemen der Träger (d.h. das Depotmaterial) aus einem SMP Material bestehen bzw. ein solches enthalten. Wird dann der SM Effekt ausgelöst, so führt die damit verbundene Formänderung dazu, dass die Membranen, die die Depots verschießen, zerstört werden, was die Freisetzung ermöglicht.

3. Bei Matrixsystemen kann durch das Auslösen den SM Effekts das Freisetzungsverhalten verändert werden, z.B. durch Veränderung der Kristallinität, der Porenstruktur oder Ähnliches.

4. Bei allen oben genannten Systemen kann allerdings das Freisetzungsverhalten auch durch den Abbau des SMP Materials gesteuert werden.

Bei allen Systemen, die SMP Materialien umfassen, können bei diesen der SM Effekt durch einen geeigneten Stimulus ausgelöst werden, wie Temperatur, Licht (Strahlung) oder eine Kombination davon. Wie oben bereits ausgeführt, können die Wirkstoffreisetzungssysteme unterschiedliche SMP Materialien umfassen, die durch unterschiedliche Stimuli beeinflußt werden können.

Wird der SM Effekt nicht für die Steuerung der Wirkstoffreisetzung benötigt, so kann er zur Herstellung von Implantaten dienen, die minimalinvasiv eingeführt werden können. Dabei wird das Wirkstoffreisetzungssystem mit Wirkstoff beladen und in eine Form gebracht, die eine minimalinvasive Implantation ermöglicht, wobei dieses Form im Hinblick auf das SMP Material der temporären Form entspricht. Nach Impantation wird der SM Effekt ausgelöst (s.o.) und das Implantat wird in die permanente Form (im Hinblick auf die SM Eigenschaften) gebracht, die in diesen Fällen üblicherweise sperriger ist als die temporäre Form.

Der in der vorliegenden Anmeldung eingesetzte Begriff Wirkstoff, wird in einem breiten Sinn verwendet. Dieser Begriff soll sowohl chemische als auch biologische Substanzen oder Gemische umfassen, die im weitesten Sinne als Wirkstoff aufgefasst werden können. Insbesondere mit umfasst sind Substanzen oder Gemische mit medizinischer Wirkung, als Medikament, aber auch als Diagnostikum oder als Kontrastmittel. Weiterhin sollen auch kosmetisch wirksame Substanzen und Gemische mit umfasst sein, ebenso wie Hilfsstoffe, wie künstliche Tränen oder Ähnliches. Der oder die in der vorliegenden Erfindung einsezubaren Wirkstoffe können niedermolekular oder hochmolekular (z.B. Proteine) sein.

Der Ausdruck Wirkstoffreisetzungssystem, so wie er in der vorliegenden Anmeldung eingesetzt wird, umfasst die zwei bereits oben geschilderten grundlegende Typen an Systemen.

Der erste Grundtyp umfasst eine Matrix, in der der Wirkstoff, der freigesetzt werden soll, verteilt vorliegt. Solche System werden insbesondere als Implantate zur Freisetzung von Medikamenten über einen verlängerten Zeitraum eingesetzt. Hierbei hat es sich erfindungsgemäß gezeigt, dass Beladungen von 1 bis 25 Gew.-% Wirkstoff möglich sind, ohne dass ein nachteiliger Effekt auf die Form-Gedächtnis-Eigenschaften ausgeübt wird.

Der zweite Grundtyp ist von seinem Aufbau etwas komplexer und umfasst ein Depot an Wirkstoff und zusätzlich eine Konstruktion, die die Freisetzung regelt, beispielsweise eine Membran, die das Depot an Wirkstoff umgibt, oder ein osmotisches Pumpsystem (siehe insbesondere: K.Heilmann, "Therapeutische Systeme", Ferdinand Enke Verlag, Stuttgart, 1882; sowie WO 99/62576).

Die erfindungsgemäßen Freisetzungssysteme können in einer Vielzahl von Indikationsbereichen eingesetzt werden, insbesondere aber zur Behandlung und/oder Prophylaxe von Störungen (Krankheiten, Allergien, postoperative Beschwerden), die eine langanhaltende Wirkstofffreisetzung erfordern, um Schmerzen zu mildern, um Geweberegeneration zu unterstützen, um Infektionsschutz zu bieten oder um Infektionen zu bekämpfen.

Neben den im folgenden beschriebenen wesentlichen Bestandteilen der Wirkstofffreisetzungssysteme können diese weitere Komponenten aufweisen, wie Beschichtungen, Additive usw., die beispielsweise die Bioverträglichkeit (Gewebeverträglichkeit) einstellen oder andere Eigenschaften, wie Röntgenkontrast usw.

Die vorliegenden Erfindung zeichnet sich dadurch aus, dass die Formgedächtnispolymere (im folgenden auch SMP (shape memory polymer)) als wesentliche Komponente mit umfasst sind, d.h. entweder als wesentlicher Bestandteil der Matrix (beim ersten Grundtyp) oder als wesentlicher Bestandteil der Membran oder der osmotischen Pumpe (im zweiten Grundtyp). Der Einsatz von SMP Materialien ermöglicht hierbei, dass der Formgedächtniseffekt die Wirkstofffreisetzung verbessert, wie im folgenden gezeigt werden wird.

Überraschender Weise hat es sich gezeigt, dass bestimmte bioabbaubare oder biokompatible Polymere mit Formgedächtniseigenschaften als Matrixmaterialien für Wirkstofffreisetzungssysteme sowie als Bestandteil der anderen Wirkstoffreisetzungssysteme geeignet sind. Die erfindungsgemäß einsetzbaren polymeren Materialien lassen sich grob in zwei Klassen unterteilen, zum einen thermoplastische Polymere und andererseits Thermoset-Polymere.

Prinzipiell sind dabei alle SMP Materialien einsetzbar, die z.B. in den beiden Internationalen Patentanmeldungen WO 99/42528 und WO 99/42147 offenbart sind. Die Offenbarungen dieser beiden Anmeldungen sind in dieser Beziehung hier durch Verweis mit umfasst. Derartige Materialien können in der Form von thermoplastischen Materialien oder in der Form von Netzwerken vorliegen. Diese Form-Gedächtnis-Polymere, die erfindungsgemäß eingesetzt werden können, können eine oder auch zwei Formen im Gedächtnis haben und umfassen dabei mindestens ein Hartsegment und mindestens ein Weichsegment. Die Struktur der Polymere ist nicht beschränkt und geeignete Beispiele umfassen lineare Polymere, Pfropfpolymere, Dendrimere, verzweigte Polymere, sternförmige Polymere (für thermoplastische Materialien) und semi-interpenetrierende Netzwerke, interpenetrierende Netzwerke, gemischte interpenetrierende Netzwerke und Netzwerke (Thermoset-Materialien).

Diese Strukturen umfassen üblicherweise Segmente abgeleitet von Caprolacton, Paradioxanon, Lactid, Glycolid oder Ethylen- oder Propylenoxidoligomeren. Im Fall der thermoplastischen Materialien kann dabei die Verknüpfung der einzelnen Segmente (Oligomere/Makromonomere) in Diolform durch ein Diisocyanat erfolgen, beispielsweise TMDI. Die Netzwerkstrukturen können in der Form von Segmenten mit Acrylatendgruppen vernetzt werden, ggf. unter Zusatz niedermolekularer Acrylate. Werden sternförmige Makromonomere eingesetzt, d.h. Makromonomere mit mehr als zwei Enden, so kann die Vernetzung auch über OH-Endgruppen erfolgen, z.B. unter Einsatz von Diisicyanaten, wie TMDI.

Bevorzugte Materialien für die erfindungsgemäßen Wirkstofffreisetzungssysteme sind allerdings wir folgt:

Die thermoplastischen Polymere, die in der vorliegenden Erfindung eingesetzt werden können, können als Blockcopolymere beschrieben werden, umfassend jeweils mindestens ein Hartsegment und mindestens ein Weichsegment, wobei das Hartsegment Einheiten umfasst, abgeleitet von Paradioxanon, und wobei das Weichsegment Einheiten umfasst, abgeleitet von Caprolacton und/oder Lactid und Glycolid. Die jeweiligen Segmente werden bevorzugt über Urethanbindungen miteinander verknüpft. Die einzelnen Segmente weisen bevorzugt ein Zahlenmittel des Molgewichts von 1000 bis 10000 g/mol auf, insbesondere bevorzugt von 3000 bis 8000 g/mol. Die Verknüpfung erfolgt bevorzugt über Urethanbindungen, erhalten durch Reaktion geeignet funktionalisierter Segmente mit TMDI. Das Molgewicht der enstehenden thermoplastischen Polymere ist nicht kritisch und liegt im üblichen Bereich für derartige SMP-Materialien.

Die erfindungsgemäß einsetzbaren Thermoset-Materialien sind Netzwerke, die semi-kristallin oder amorph sein können. Die bevorzugten, erfindungsgemäß einsetzbaren Netzwerke sind Polyurethannetzwerke, die durch die Vernetzung von geeignet funktionalisierten Makromonomeren erhalten werden können und diese umfassen bevorzugt Segmente aus Caprolacton, Glykolid, Lactid und/oder Dioxanon.

Die semikristallinen Thermoset-Materialien umfassen bevorzugt eine Komponente, abgeleitet von einem Makromonomer aus Caprolacton und Glycolid. Die amorphen Netzwerke umfassen Komponenten, abgeleitet von Makromonomeren aus Lactid und Glycolid, Capolacton und Lactid oder Lactid und Dioxanon. Die Netzwerkstrukturen können darüber hinaus gegebenenfalls noch weitere optionale Netzwerkbestandteile aufweisen, wobei diese zusätzlichen Bestandteile vorzugsweise ausgewählt sind unter Acrylaten und Methacrylaten, insbesondere bevorzugt Butylacrylat.

Auch hier beträgt das bevorzugte Zahlenmittel des Molgewichts für die Segmente von 1000 bis 10000 g/mol,

insbesondere 3000 bis 10000 g/mol. Liegen mehrere Monomereinheiten in den Segmenten vor, z.B. Lactid- und Glykolid-Einheiten oder Caprolacton- und Glykolid-Einheiten, so ist deren jeweiliger Anteil nicht beschränkt. Bevorzugt liegt in solchen Fällen jedoch Glykolid in einem Anteil von mehr als 0 bis zu 30 Mol% vor, bevorzugt in einem Anteil von 10 bis 20 Mol%.

Wird bei der Vernetzung noch ein Acrylatmonomer zugesetzt, so liegt dieses bevorzugt in einem Anteil von bis zu 60 Gew.-% vor, insbesondere 25 bis 55 Gew.-%.

Die oben genannten Materialien ermöglichen üblicherweise eine Auslösung des Form-Gedächtnis-Effekts durch einen Temperaturstimulus, aber die SMP-Materialien können auch so ausgestaltet werden, dass sie durch einen anderen Stimulus gesteuert werden können, wie Magnetfetder, Ultraschall, Licht, Elektrizität oder andere Stimuli.

Die oben genannten polymeren Materialien eignen sich insbesondere als Matrixmaterialien für Wirkstofffreisetzungssysteme. Die bevorzugten Matrixmaterialien sind bioabbaubar, so dass insbesondere bei einer Wirkstofffreisetzung im Körper eine zweite Operation zur Entfernung der Matrix nach erfolgter Wirkstofffreigabe nicht notwendig ist. Weiterhin zeigen die erfindungsgemäß einzusetzenden Matrixmaterialien ein Abbauverhalten, das nicht mit einer drastischen Abnahme der mechanischen Eigenschaften der Matrixmaterialien verbunden ist. Weiterhin sind die erfindungsgemäß eingesetzten Matrixmaterialien Polymere, die Formgedächtniseigenschaften aufweisen.

Der Ausdruck "Polymere mit Formgedächtniseigenschaften", der in der vorliegenden Erfindung verwendet wird, benennt Materialien, die von der vorgegebenen permanten Gestalt in eine temporäre Form überführt werden können (durch geeignete Formungsverfahren) und nach Anlegen eines externen Stimulus wieder in die permanente Form übergehen. Die Deformation und Fixierung der temporären Form wird Programmierung genannt. Der Übergang von der temporären in die permanente Gestalt wird als Rückstellung bezeichnet. Die Initiierung der Rückstellung erfolgt bei den in der vorliegenden Erfindung eingesetzten Matrixmaterialien üblicherweise durch thermische Stimulation.

Die Formgedächtniseigenschaften der in der vorliegenden Erfindung eingesetzten Matrixmaterialien ermöglicht zum einen, dass bei zu implantierenden Wirkstofffreisetzungssystemen eine temporäre Form fixiert werden kann, die minimalinvasive Eingriffe ermöglicht. Nach Applikation am gewünschten Zielort kann durch geeignete Stimulierung, üblicherweise Temperaturerhöhung auf Grund der Körpertemperatur, ein Formgedächtniseffekt aufgelöst werden. Dieser Formgedächtniseffekt kann dann dazu genutzt werden, dass der im Wirkstofffreisetzungssystem eingeschlossene Wirkstoff freigesetzt wird. Dabei lassen sich verschiedene Aufgestaltungen einstellen.

Zum einen kann durch den Formgedächtniseffekt eine Veränderung der Phasenstruktur, der Porenstruktur, der Oberflächenstruktur oder der Kristallinität erreicht werden, was dann eine langsame und gleichmäßige Freisetzung des Wirkstoffes ermöglicht. Derartige Matrixsysteme können aber optionale zusätzliche Schichten aufweisen, so kann beispielsweise die mit Wirkstoff beladene Matrix von einer Schicht eines nicht-SMP-Materials umgeben sein (coreshell-system). Dieses zusätzliche Material kann z.B. eine sicherere Einführung des Wirkstofffreistzungssytems ermöglichen, z.b. durch entsprechende Oberflächenausgestaltung. Diese Beschichtungsschicht kann bioabbaubar ausgestaltet sein, so dass nach Applikation am gewünschten Wirkort die Beschichtung zersetzt wird und die gewünschte Feisetzung durch das SMP-Material ermöglicht wird. Alternativ kann die zusätzliche Beschichtung selbst Wirkstoffe enthalten, die vor den Wirkstoffen freigesetzt werden, die in der SMP-Matrix eingelagert sind. So ist auch eine Kombinationstherapie mit zeitlich abgestimmter Reihenfolge der Freisetzung möglich. Eine weitere Möglichkeit des Einsatzes einer zusätzlichen Beschichtung ist dann ratsam, wenn das SMP-Material eine sehr schnelle Freisetzung nach Auslösung des Formgedächtniseffekts und eine schon merkliche Freisetzung vorher zeigt. Die zusätzliche Beschichtung verhindert somit eine nicht erwünschte Wirkstofffreisetzung vor dem Auslösen des Formgedächtniseffekts. Dieser kann dann gezielt ausgelöst werden, wobei Ausgestaltungen denkbar sind, bei denen durch den Form-Gedächtnis-Effekt die äußere Beschichtung zerstört und dann eine sehr schnelle, gewünschte Freisetzung erfolgt.

Weiterhin kann bei Matrixsystemen die Freisetzung durch Diffusion (Kristallinität der Matrix), durch Abbaureaktionen (Zersetzung der Matrix setzt den Wirkstoff frei) oder eine Kombination davon gesteuert werden. Beispielsweise kann eine Matrix mehrere unterschiedliche Domänen aufweisen, von denen mindestens eine eine gezielte Änderung des Diffusionskoeffizienten, der Tm oder Tg zeigt, so dass derart eine Kontrolle der Freisetzung möglich wird. Eine weitere Option der Steuerung der Freisetzungsrate aus einer mit Wirkstoff beladenen Matrix ist die geeignete Auswahl der Verträglichkeit von Wirkstoff und Matrixmaterial (SMP Material). Eine gute Verträglichkeit führt per se zu einer langsameren Verträglichkeit, verglichen mit einem System, bei der eine geringere Verträglichkeit zwischen Wirkstoff und Matrixmaterial gegeben ist. So führt beispielsweise die Erhöhung des hydrophoben (oder auch amorphen Anteils) Anteils in einem Netzwerk zu einer geringeren Freisetzungsrate von hydrophoben Wirkstoffen. Eine derartige Erhöhung des hydrophoben Anteils eines Netzwerks (oder auch eines thermoplastischen Materials) kann insbesondere durch die Einführung von Butylacrylatsegmenten erfolgen. Insbesondere bei Wirkstofffreisetzungssytemen mit Wirkstoffen wie Ethacridinlactat hat die Erhöhung des Anteils an Glykolat in der Matrix, was die Kristallinität verringert, einen beschleunigenden Effekt auf die Wirkstoffreisetzung. Durch geeignete Einstellung der jeweiligen Anteile kann also eine gewünschte Wirkstofffreisetzung gezielt eingestellt werden.

Derartige Matrixsysteme können in vielen Ausgestaltungen vorliegen, die vereinfacht als 3D-, 2D- oder 1 D-Systeme bezeichnet werden können, wie Kügelchen oder kastenartige oder zylindrische Implantate (3D), Filme oder

Folien, ungestreckt oder verstreckt (2D), oder Fäden und Filamente (1 D). Derartige Matrixsysteme könne darüber hinaus zu interessanten, komplexen Systemen zusammengesetzt werden, so können beispielsweise Matrixfilme mit anderen, nicht mit Wirkstoff beladenen Filmen zu mehrlagigen Laminaten zusammen gesetzt werden, um dadurch eine weitere Kontrolle der Wirkstofffreisetzung zu ermöglichen. Derartige Laminatsysteme umfassen bevorzugt n Filme aus mit Wirkstoff beladenem SMP-Material und immer alternierend damit vorgesehene (n + 1) Filme aus einem nicht mit Wirkstoff beladenem Film, der aus SMP-Material oder einem nicht SMP-Material bestehen kann. Dieses Prinzip ist auch bei anderen Matrixsystemen vom 1D oder 3D Typ möglich. So kann beispielsweise ein mit Wirkstoff beladenes 3D oder 1D System mit einer zusätzlichen Beschichtung aus SMP-Material, ohne Wirkstoff, umgeben sein, z.B. um die Freisetzungsrate zu kontrollieren.

Die Einführung der Wirkstoffe in solche Matrixsystem kann auf unterschiedliche Arten erfolgen, in Abhängigkeit vom Wirkstoff und vom SMP-Material. Geeignete Verfahren umfassen (a) das gemeinsame Lösen von Wirkstoff und SMP-Material in einem Lösungsmittel und anschließende Trocknung (oder alternativ Ausfällung mit einem Nichtlösungsmittel für beide Stoffe), (b) die Vermischung von Wirkstoff und einem Vorläufermaterial für ein SMP-Material und anschließende Vernetzung des Vorläufermaterials (ggf. erfolgt die Vermischung in einem Lösungsmittel, das dann entfernt wird), oder (c) Quellen von Objekten aus SMP-Materialien in einer Lösung des Wirkstoffs. Weitere Möglichkeiten der Einbringung des Wirkstoffs in eine Matrix umfassen die Schmelzvermischung (z.B. in einem Extruder), die chemische Fixierung des Wirkstoffs an die Matrixmoleküle oder Ähnliches.

Andererseits ist es auch möglich, den Formgedächtniseffekt in Freisetzungssystemen des zweiten Grundtyps einzusetzen. Auch hierbei kann erreicht werden, dass ein eingeschlossener Wirkstoff über einen kurzen Zeitraum freigesetzt wird, was in manchen Anwendungsbereichen erwünscht ist. In diesem Fall kann das Matrixmaterial des Wirkstofffreisetzungssystems der vorliegenden Erfindung die Funktion einer Membran haben, die nach Initiierung des Formgedächtniseffekts vollständig durchlässig für den eingeschlossenen Wirkstoff wird, so dass eine schlagartige Freisetzung möglich ist. Derartige Wirkstofffreisetzungssysteme können entweder insgesomt aus einem SMP-Material bestehen, z.B. in der Form eines Hohlkörpers, der ein Depot an Wirkstoff einschließt, oder dieses System besteht aus einem Depotsystem für den Wirkstoff mit einer Öffnung, die durch das SMP-Material verschlossen ist.

Die erste Alternative bietet sich insbesondere für Einkapselungssysteme an, z.B. für topisch zu verwendende Wirkstoffe. Im Bereich der Kosmetik können so Wirkstoffe, wie Vitamine, Hautpflegemittel oder andere, ggf. oxidationsanfällige Stoffe eingekapselt werden, die dann bei Aufbringung auf die Haut, durch Wärmeeinwirkung einen Form-Gedächtnis-Effelt zeigen und die eingeschlossenen Wirkstoffe freisetzen. Andere Anwendungsbeispiele sind Reservoirs für Künstliche Tränen oder eingekapselte topisch zu verabreichende Medikamente.

Hierbei kann der Formgedächtniseffekt der erfindungsgemäßen Wirkstofffreisetzungssysteme, insbesondere bei Wirkstofffreisetzungssystemen die topisch verabreicht werden, dazu dienen, den eingeschlossenen Wirkstoff kontrolliert freizusetzen. Beispiele derartiger Ausgestaltungen der erfindungsgemäßen Wirkstofffreisetzungssysteme sind Trägerkapseln für Hautpflegemittel oder Trägerkapseln für Wirkstoffe, die intraaural, intranasal oder mukosal verabreicht werden. Dabei sind die Trägerkapseln, die die erfindungsgemäßen Wirkstofffreisetzungssysteme darstellen, in üblichen Formulierungen für topisch zu verabreichende Wirkstoffe formuliert. Ein derartiges Beispiel ist eine Hautcreme, in der bestimmte pflegende Substanzen in erfindungsgemäßen Wirkstofffreisetzungssystemen eingeschlossen sind. Dies bietet den Vorteil, dass insbesondere bei Formulierungen, die über einen langen Zeitraum angewandt werden, eine Beeinträchtigung der Wirkstoffe, beispielsweise durch Luftsauerstoff vermieden oder zumindest stark verzögert werden kann. Nach der üblichen Verabreichung kann dann eine gezielte Wirkstofffreisetzung erfolgen, beispielsweise durch die Auflösung eines temperaturinduzierten Formgedächtniseffekts nach Verabreichung der Formulierung (beispielsweise Auftragen einer Hautcreme auf die Haut, wobei der Wirkstoff auf Grund eines thermischeninduzierten Formgedächtniseffekts freigesetzt wird, nachdem die erfindungsgemäßen Wirkstoffsysteme einige Zeit auf der Haut vorliegen und dort auf eine Temperatur in der Nähe der Körpertemperatur erwärmt wurden).

Die im erfindungsgemäßen Wirkstofffreisetzungssystem einzuschließenden Wirkstoffe können ausgewählt werden aus einer Vielzahl an Wirkstoffen. Dabei umfasst der Ausdruck Wirkstoff sowohl Arzneimittel als auch andere Wirkstoffe, wie Hautpflegemittel, künstliche Tränen, Geruchsstoffe, Substanzen die zur Diagnostik notwendig sind, wie Kontrastmittel oder radioaktive Labels, und ähnliches. Bevorzugte Wirkstoffe umfassen Hormone, Antibiotika, Enzyme, Antikrebsmittel, Peptide, Anesthetika, Psychopharmaka, Analgetika, Antiseptika, Antimykotika, Antihistaminika, Virustatika und Wachstumsfaktoren. Wirkstoffe, die bereits erfolgreich in Freisetzungssystemen mit SMP-Materialien eingesetzt wurden umfassen Ethacridinlactat, Enoxacin, Nitrofurantoin und Gentamicin.

Derartige Wirkstoffe lassen sich ohne große Probleme in die erfindungsgemäß verwendeten polymeren Materialien einschließen. Bei Wirkstofffreisetzungssystemen, bei denen das polymere Material als Matrix dient, können die Wirkstoffe übliche Verfahren eingebracht werden. Bei den thermoplastischen Materialien können die Wirkstoffe durch Dispersion der Wirkstoffe in einer Polymerlösung und anschließendes Trocknen eingeschlossen werden. Anschließend kann die getrocknete Mischung einem Programmierungsschritt unterworfen werden, um so die gewünschten Formgedächtniseigenschaften für die Wirkstofffreisetzung zu erzielen. Gegebenenfalls können anschließend weitere Verarbeitungsschritte erfolgen, beispielsweise Zerkleinerungsschritte oder Formulierungsschritte, wobei diese Schritte so ausgeführt werden müssen, dass eine unbeabsichtigte Initiierung des Formgedächtniseffekts vermieden wird. Die erfindungsgemäß einzusetzenden Thermoset-Materialien können mit Wirkstoffen entweder durch Quellung in einer Wirkstofflösung beladen werden oder durch ein Verfahren, bei dem die löslichen Vorläufermaterialien der Thermoset-Materialien zusammen mit dem Wirkstoff in einer Lösung vorliegen, wobei durch anschließendes Entfernen des Lösungsmittels und darauf folgende Vernetzung ein beladenes Thermoset-Material erhalten wird. Alternativ ist es auch möglich die Mischung aus Präpolymer und Wikstoff ohne Lösungsmittel herzustellen, so dass der Wirkstoff dann dispergiert in der polymerisierbaren Mischung vorliegt. Anschließen kann auch hier eine erwünschte Programmierung erfolgen, gefolgt wiederum von weiteren optionalen Verarbeitungsschritten.

Bei der Einbringung von Wirkstoff in ein Wirkstofffreisetzungssystem in Übereinstimmung mit der vorliegenden Erfindung können somit die folgenden prinzipiellen Fälle unterschieden werden:

1. Der Wirkstoff kann entweder niedermolekular oder hochmolekular sein.
2. Der Wirkstoff kann hydrophil (polar) oder hydrophob (unpolar) sein.

Für diese prinzipiellen Fälle lassen sich die folgenden, allgemeinen Regeln zur Wirkstoffbeladung aufstellen.

<u>Niedermolekularer hydrophiler Wirkstoff (z.B. Ethacridinlactat)</u>

Quellung von Netzwerken in einer geeigneten Wirkstofflösung (hydrophile Lösungsmittel, wie Propanol, Ethylacetat, um den Wirkstoff zu lösen) und anschließende Trocknung (Beladung typischer Weise von 0,1 bis ungefähr 2 Massen-%)

Vernetzung von Präpolymeren in der Gegenwart von Wirkstoff (alternativ gelöst in einem geeigneten Lösungsmittelsystem oder dispergiert in der polymerisierbaren Mischung). Eine derartige Einbringung ist für hydrophile Wirkstoffe gut geeignet, da anschließende Verarbeitungsschritte mit hydrophoben Lösungsmitteln die Wirkstoffbeladung nicht mehr beeinträchtigen (Beladung typischer Weise bis zu 6 Massen-%).

<u>Niedermolekularer hydrophober Wirkstoff (z.B. Enoxacin Nitrofurantoin)</u>

Quellung von Netzwerken in einer geeigneten Wirkstofflösung (eher hydrophobere Lösungsmittel, wie Dioxan, Chloroform, aber auch Ethylacetat, um den Wirkstoff zu lösen) und anschließende Trocknung (Beladung typischer Weise von 0,1 bis ungefähr 2 Massen-%)

Vernetzung von Präpolymeren in der Gegenwart von Wirkstoff (alternativ gelöst in einem geeigneten Lösungsmittelsystem oder dispergiert in der polymerisierbaren Mischung) (Beladung typischer Weise bis zu 6 Massen-%).

<u>Hochmolekularer Wirkstoff</u>

Auch hochmolekulare Wirkstoffe, wie Proteine, können in die erfindungsgemäßen Wirkstofffreisetzungssysteme eingearbeitet werden. Allerdings ergibt sich dabei häufig das Problem, dass hochmolekulare Wirkstoffe zum Einen durch Quellverfahren nur in nicht ausreichenden Mengen in die Netzwerke eingebracht werden können und zum Anderen die übliche Verarbeitung von thermoplastischen Systemen zu mechanischen oder thermischen Beanspruchungen führen, die für derartige hochmolekulare Wirkstoffe nachteilig sind. Daher werden derartige Wirkstoffe bevorzugt erst durch Einkapselung, beispielsweise in PEG-Mikropartikel, oder durch Wechselwirkung mit einem Polyelektrolyt geschützt/ stabilisiert, bevor eine Einbringung in eine Matrix erfolgt. Diese Einbringung erfolgt dann bevorzugt durch Netzwerkherstellung in der Gegenwart des Wirkstoffs durch Vernetzung von Präpolymeren oder durch traditionelle Verarbeitung für thermoplastische Materalien, wie Extrusion.

Überraschenderweise hat sich gezeigt, dass die anspruchsvollen Materialien, die in der vorliegenden Erfindung als Matrixmaterialien für die Wirkstofffreisetzungssysteme eingesetzt werden, in ihren thermischen und mechanischen Eigenschaften durch die Beladung mit Wirkstoffen praktisch nicht beeinflusst werden. So verändert sich beispielsweise die Schalttemperatur (Auslösungstemperatur für den Formgedächtniseffekt) bei beladenen Wirkstofffreisetzungssystemen im Vergleich mit den unbeladenen Wirkstofffreisetzungssystemen nicht oder nur unwesentlich. Auch die mechanischen Eigenschaften, wie E-Modul, werden durch die Beladung mit Wirkstoffen nicht oder nur geringfügig beeinflusst. Daher können die erfindungsgemäßen Wirkstofffreisetzungssysteme trotz der Beladung mit Wirkstoff den Formgedächtniseffekt zur Wirkstofffreisetzung zeigen.

Allerdings findet bei einigen Systemen eine leichte Veränderung z.B. von Tg oder Tm, was ggf. auf "Weichmachereffekte" durch niedermolekulare Wirkstoffe zurück zu führen ist oder auf die (teilweise) Unterdrückung der Kristallisation. Derartige Effekte treten allerdings in einem merklichen Umfang lediglich bei Homonetzwerken auf, z.B. Netzwerken auf Basis von Caprolacton und insbesondere wenn die Beladung durch Netzwerkherstellung in der Gegenwart von dispergiertem Wirkstoff bei geringen Segmentlängen der Präpolymere erfolgt. Bei AB-Netzwerken werden

derartige Effekte eher nicht beobachtet, so dass durch Veranderung der Netzwerkarchitektur eine Veränderung der thermischen Eigenschaften unterdrückt werden kann.

Insgesamt ergibt sich so ein interessantes und vielseitiges System, das je nach Anwendungsgebiet an die speziellen Anforderungen angepasst werden kann.

Eine weitere Möglichkeit besteht darin, dass die oben als Matrixmaterialien beschriebenen Polymere mit Formgedächtniseigenschaften ein Wirkstoffdepot umhüllen, also selbst nicht den eingeschlossenen Wirkstoff enthalten (oder nur in einem sehr geringen Anteil). Derartige Einkapselungssysteme sind insbesondere dann von Vorteil, wenn die Verträglichkeit zwischen Wirkstoff und Polymer mit Formgedächtniseigenschaften gering ist. Derartige Einkapselungssysteme können in üblicher Art und Weise hergestellt werden. Dadurch, dass diese Ausgestaltung der erfindungsgemäßen Wirkstofffreisetzungssysteme die erfindungsgemäß eingesetzten Polymere mit Formgedächtniseigenschaften lediglich als Umhüllungsmaterial verwendet, kann durch die geeignete Auswahl dieses Materials das Freisetzungsverhalten gezielt gesteuert werden. Zum einen ist es möglich, ein Material auszuwählen, das nach Auslösung des Formgedächtniseffekts vollständig durchlässig für den eingeschlossenen Wirkstoff ist, so dass hier eine sehr schnelle Wirkstofffreisetzung erreicht wird. Alternativ kann ein Material ausgewählt werden, das nach Auslösung des Formgedächtniseffekts nur einen sehr langsamen Austritt des Wirkstoffs ermöglicht, so dass in dieser Ausführungsform eine lang andauernde und kontrollierte Wirkstofffreisetzung erhalten werden kann.

Selbstverständlich ist es bei den erfindungsgemäßen Wirkstofffreisetzungssystemen auch möglich, den Formgedächtniseffekt nicht für die Wirkstofffreisetzung einzusetzen, sondern lediglich für die Platzierung von Implantaten durch minimalinvasive Eingriffe. Dadurch, dass die bevorzugten, erfindungsgemäß eingesetzten Polymere bioabbaubar sind, wird eine vergleichsweise gleichmäßige Wirkstofffreisetzung, insbesondere bei matrixartigen Wirkstofffreisetzungssystemen durch den langsamen Abbau im biologischen System erhalten. Da die erfindungsgemäßen Wirkstofffreisetzungssysteme ein Abbauverhalten zeigen, das nicht von nachteiligen Effekten wie dem burst-Effekt begleitet ist, ermöglicht eine derartige Ausgestaltung der erfindungsgemäßen Wirkstofffreisetzungssysteme auch ohne Ausnützung des Formgedächtniseffekts eine kontrollierte Wirkstofffreisetzung.

Durch die universelle Einsdtzbarkeit der SMP Materialien und durch die oben geschilderten Variationsmöglichkeiten erstreckten sich bevorzugte Anwendungsgebiete der erfindungsgemäßen Wirkstofffreisetzungssysteme über einen weiten Bereich.

So können die Wirkstoffreisetzungssysteme Bestandteil von Implantaten sein. Dies ist entweder durch eine (teilweise) Beschichtung der Implantate möglich oder dadurch, dass die Implantatae selbst ein geeignetes SMP Material zur Wirkstofffreisetzung umfassen. Beispile solcher Systeme sind Stents, Gelenkprothesen, Gefäßprothesen, Nahtmaterialien, chirurgische Hilfsmittel, wie Klammern, Katheter, Nadeln von Spritzen und vieles mehr. Insbesondere Stents, Nahtmaterial oder Gefäßstützen können selbst das SMP Material umfassen, während die anderen Beispiele bevorzugt eine (teilweise) Beschichtung aufweisen. Durch eine derartige Ausgestaltung ist es möglich, dass die Implantate bzw. die anderen Gegenstände zusätzlich zu ihrer eigentlichen Funktion noch als Wirkstoffreisetzungssystem dienen. Dies bietet große Vorteile, da beispielsweise notwendige Wirkstoffe direkt über das Implantat bzw. den Gegenstand zur Verfügung gestellt werden. Prothesen können Beschichtungen aufweisen, die Wirkstoffe freisetzen, benötigt um Abstoßungsreaktionen oder Entzündungen zu verhindern. Nahtmaterialien können ebenfalls entzündungshemmende Wirkstoffe freisetzen, während Stents mit Wirkstoffen ausgestattet sein können, die z. B. Blutgerinnung verhindern oder Besiedelung der Stents mit Zellen.

Eine weitere Alternative ist es die erfindungsgemäßen Wirkstofffreisetzungssysteme in Partikelform einzusetzen, beispielsweise in der Form von Mikro- oder Nanopartikeln (Matrixsystem) oder in der Form von Mikro- oder Nanokapseln. Derartige Partikel sind in einer Vielzahl von Anwendungsbereichen hilfreich, von denen im folgenden einige geschildert werden.

Mikropartikel könne insbesondere auch für Anwendungen im Auge verwendet werden, entweder als Wirkstoffträger allein oder mit einer zusätzlichen Funktion. So können solche Mikropartikel dazu dienen den Tränenkanal temporär zu schließen, was für einige Therapien erforderlich ist. Dort wird dann auch der Wirkstoff freigesetzt. Der Tränenkanal wird dann nach einer gewünschten Zeitdauer wieder frei. Mikropartikel können weiter als Wirkstoffträger oder zur Okklusionstherapie in der Blutbahn Anwendung finden.

Erfindungsgemäße Partikel können weiterhin in Aerosolen eingesetzt werden, zur pulmonalen Wirkstofffreisetzung.

Schließlich können Partikel auch im Bereich Tissue Engineering eingesetzt werden, um on-demand bioaktive Substanzen aus der das Gewebe tragenden Struktur freizusetzen.

Die erfindungsgemäßen Wirkstofffreisetzungssysteme können aber auch in transdermalen Anwendungen eingesetzt werden, wie Pflastern. Durch den SM Effekt kann bei solchen Systemen der Diffusionskoeffizient gezielt verändert werden, um eine transdermale Wirkstoffverabreichung zu steuern, beispielsweise durch Erhöhung der Freisetzung des Wirkstoffs.

Es sind auch orale Verabreichungsformen darstellbar, bei denen sich die Wirkstofffreisetzung gezielt im Magen oder Darm steuern lässt. Alternativ kann bei solchen Systemen das vorstehend beschriebene Prinzip der Wirkstoffpumpe

zum Einsatz kommen.

Im folgenden werden bevorzugte, erfindungsgemäß einzusetzenden Polymere näher beschrieben.

Netzwerke

Kovalente Polymernetzwerke aus Oligo(ε-hydroxycaproat)dimethacrylaten und Butylacrylat sind Formgedächtnispolymere, die sich *in vitro* als hydrolytisch abbaubar und zellkompatibel erwiesen haben. Durch eine geeignete Wahl der molekularen Parameter des Polymersystems können die Eigenschaften, wie die Schmelztemperatur, die Kristallinität, das hydrolytische Abbauverhalten und die Hydrophilie des Materials, eingestellt werden. Die Polymernetzwerke werden photochemisch nach Endgruppenfunktionalisierung der zugrunde liegenden Makrodiole zu präpolymeren Dimethacrylaten dargestellt. Zur Steuerung der Abbaugeschwindigkeit werden leicht hydrolysierbare Esterbindungen in die Makrodimethacrylate eingebaut. Zu diesem Zweck wird ε-Caprolacton mit Diglycolid copolymerisiert. Das Comonomerverhältnis der Makrodimethacrylate wird in der Form variiert, so dass eine Kristallisation von Glycolatsequenzen ausgeschlossen werden kann und somit die Schmelztemperatur und die Kristallinität ausschließlich durch die Oligo(ε-hydroxycaproat) segmente geprägt wird. Die Synthese der Cooligo(ester)diole basierend auf ε-Caprolacton und Diglycolid erfolgt initiiert durch Ethylenglycol und katalysiert durch Dibutylzinn(IV)oxid in Schmelze mittels ringöffnender Polymerisation (Gl. [1]).

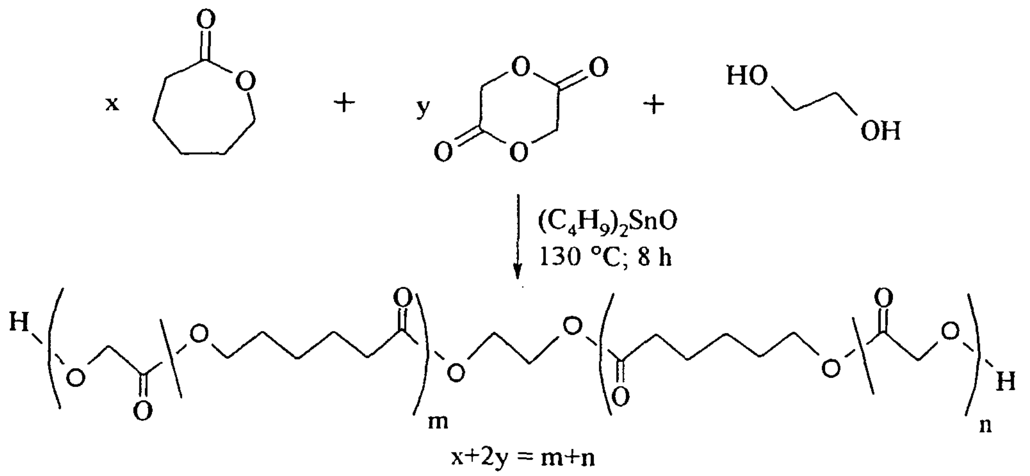


Unter Einsatz des Umesterungskatalysators Dibutylzinn(IV)oxid wird bei einer Copolymerisationsdauer von 8 h eine zufällige Verteilung der Wiederholungseinheiten im Cooligomeren erzielt. Die Endgruppenfunktionalisierung der Oligo[(ε-hydroxycaproat)-*co*-glycolat]diole basiert auf einer stöchiometrischen Umsetzung der terminalen Hydroxygruppe mit Methacryloylchlorid unter Zusatz von Triethylamin als Base (Gl. [2]).

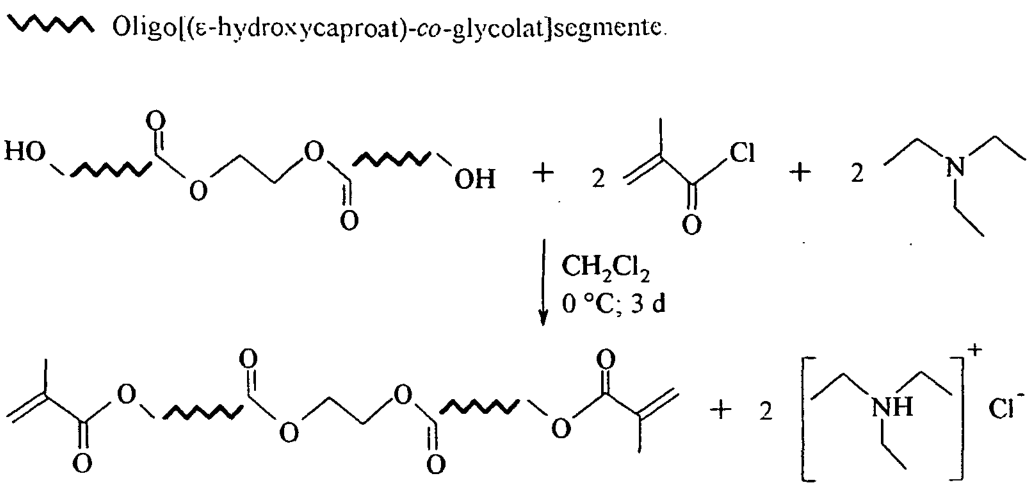

Die Präpolymere werden photoinitiiert ohne Zusatz eines Photoinitiators in der Schmelze bei 70 °C vernetzt. Bei der Darstellung der AB-Netzwerke wird Butylacrylat als Comonomer mittels Oligo[(ε-hydroxycaproat)-*co*-glycolat] dimethacrylaten vernetzt. Mit Hilfe der $^{13}$C-NMR-Spektroskopie an gequollenen AB-Netzwerken konnte nachgewiesen werden, dass der Gehalt an Butylacrylat im Netzwerk durch dessen Anteil im Reaktionsansatz kontrolliert eingestellt werden kann. Bei der Betrachtung der Netzwerkarchitektur muss zwischen den Netzwerken aus Makrodimethacrylaten und den AB-Netzwerken, die sich aus Makrodimethacrylaten und comonomerem Butylacrylat zusammensetzen, unterschieden werden.

Die Vernetzungsstellen der Netzwerke werden durch reagierende Methacrylatendgruppen der Präpolymere gebildet. Diese werden als multifunktionelle Vernetzungsstelle bezeichnet, da die Länge der dabei entstehenden Oligo (methacrylat)sequenzen in Relation zu der Segmentlänge der Cooligoester sehr viel kürzer ist. Aufgrund des Überschusses an Butylacrylat gegenüber den Oligo(ester)einheiten in den AB-Netzwerken entstehen durch die Reaktion der Dimethacrylatendgruppen mit Butylacrylat neben multifunktionellen überwiegend trifunktionelle Vernetzungsstellen.

Die Schmelztemperatur $T_m$ eines Systems bestimmt die Schalttemperatur $T_{trans}$, bei deren Überschreiten der Formgedächtniseffekt ausgelöst und die permanente Form rückgestellt wird. Im Gegensatz zu thermoplastischen Materialien wird in Polymernetzwerken die permanente Form nahezu vollständig wiedererlangt, da viskoelastische Effekte, die zu einer irreversiblen Deformation führen, durch die kovalente Vernetzung unterbunden werden.

Durch Einführung von Glycolid als Comonomereinheit der Präpolymere bis zu einem Anteil von 30 mol-% kann die Schmelztemperatur und dementsprechend $T_{trans}$ des Netzwerks zwischen 20 °C und 57 °C eingestellt werden. Die Polymerisation der Makrodimethacrylate in Gegenwart von Butylacrylat führt zu einer Senkung der Kristallinität der Materialien, die ausschließlich durch die kristallinen Bereiche der Oligo(ε-hydroxycaproat)segmente bestimmt wird. Durch das Comonomerverhältnis kann der Elastizitätsmodul der Netzwerke zwischen 0,4 MPa und 64 MPa eingestellt werden.

Amorphe Copoly(esterurethan)netzwerke aus Oligo[(rac-lactat)-co-glycolat]tetrolen und Diisocyanat bieten ebenfalls die Vorzüge einer hydrolytisch degradierbaren Polymermatrix, die über Formgedächtniseigenschaften verfügt. Copolymere bzw. Cooligomere aus rac-Dilactid und Diglycolid ermöglichen durch Variation des Comonomerverhältnisses, die Eigenschaften hinsichtlich der Glasübergangstemperatur und der Geschwindigkeit des hydrolytischen Abbaus einzustellen. Die Synthese der Polymernetzwerke erfolgt mittels Polyaddition tetrafunktioneller, hydroxytelechelischer Cooligo(ester) mit einem aliphatischen Diisocyanat.

Die tetrafunktionellen Cooligomere aus rac-Dilactid und Diglycolid werden mittels ringöffnender Polymerisation in der Schmelze dargestellt. Die Initiierung erfolgt durch Pentaerythrit in Gegenwart von Dibutylzinn(IV)oxid (Gl. [3]).

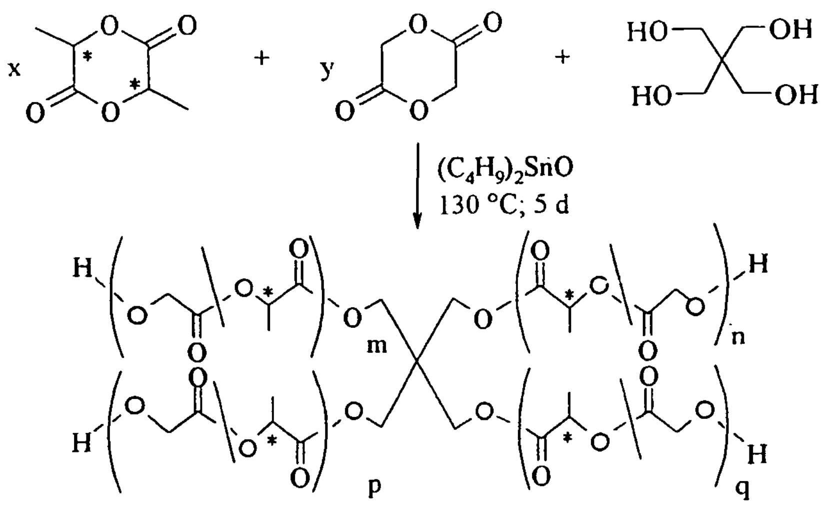


$$2(x+y) = m+n+p+q$$

Da bei der Copolymerisation von L,L-Dilactid und Diglycolid nachgewiesen wurde, dass Dibutylzinn(IV)oxid als Umesterungskatalysator fungiert, wird bei der Darstellung der Makrotetrole eine zufällige Verteilung der Wiederholungseinheiten erwartet. Die Darstellung der Copoly(esterurethan)netzwerke erfolgt durch Kupplung der tetrafunktionellen Telechele mit TMDI, gemäß Gl. [4].

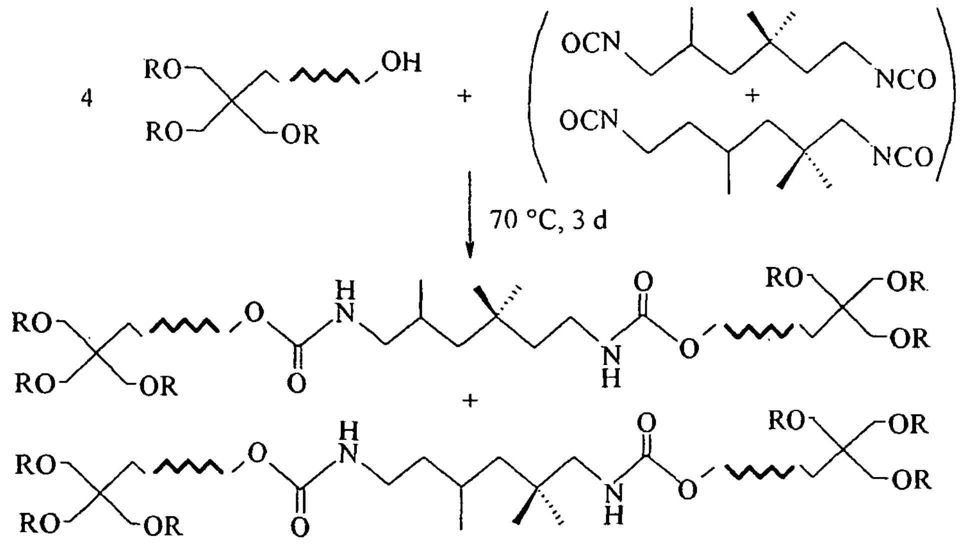


R bzw. sind die Oligo[(*rac*-lactat)-*co*-glycolat]segmente.

Um eine quantitative Kupplung der sternförmigen Hydroxytelechele zu gewährleisten und Nebenreaktionen wie Di- und Trimerisierung der Diisocyanate oder Allophanatbildung zu verhindern, muss TMDI in äquimolaren Mengen eingesetzt werden. Die erwartete Netzwerkarchitektur ist in Abb. 1 skizziert.

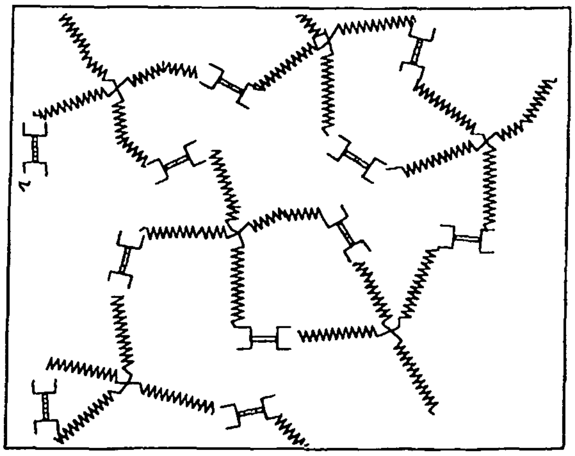

Abb. 1: Schematische Darstellung der Netzwerkarchitektur.
Oligo[(*rac*-lactat)-*co*-glycolat]segmente
Tetrafunktionelle Vernetzungsstelle
Diurethaneinheit

Durch die Verwendung tetrafunktioneller Präpolymere unter der Voraussetzung einer niedrigen Polydispersität wird ein nahezu regelmäßiges Polymernetzwerk mit definierten Vernetzungsstellen erwartet. Daher werden Netzwerke, die auf diesem Syntheseweg erhalten werden, als Modellnetzwerke bezeichnet. Lediglich die intramolekulare Kupplung zweier Kettenenden innerhalb eines Oligomers oder nicht reagierte Kettenenden (*dangling chains*) durch nicht quantitativen Umsatz können Ursache für mögliche Fehlstellen sein.

Durch die oben genannten Initiatoren, wie Ethylenglycol, Pentaerythrit oder aber auch 1,1,1-Tris(hydroxymethyl)ethan ist eine Erzeugung von mehrfunktionellen Makromonomeren möglich, d.h. lineare, dreiarmige oder vierarmige hydroxytlechelische Makromonomere.

Weitere Netzwerke, die sich auf ähnliche Weise herstellen lassen umfassen Copolyestersegmente auf Basis von Lactid und Caprolacton oder Lactid und Dioxanon, die wie oben beschrieben hergestellt werden können. Der Dioxanongehalt bz. Caprolactongehalt beträgt dabei vorzugsweise 5 bis 70 Mol% bzw. 3 bis 45 Mol%, insbesondere 10 bis 50 Mol% bzw. 10 bis 30 Mol%. Das Zahlenmittel der Segmente (Makromonomere) ist wie oben definiert. Auch hier können wieder Acrylatmonomere mit einpolymerisiert werden.

Ein weiteres erfindungsgemäß einsetzbares System ist ein Copolyester auf Basis eines Oligopropylenglycols, mit einem Zahlenmitter des Molgewichts von 1000 bis 6000 g/mol, mit Einheiten auf Grundlage von Glykolid und Lactid, so dass das Makromonomer ein Zahlenmittel des Molgewichts von ungefähr 2000 bis 15000 g/mol aufweist.

Weitere bevorzugte, im Rahmen der vorliegendenErfindung einsetzbare Netzwerke sind interpenetrierende Netzwerke.

Bevorzugte interpenetrierende Polymernetzwerke sind solche, die neben Domänen der Schaltsegmente aus Oligo[(rac-lactat)-co-glykolat] eine bei Raumtemperatur kautschukelastische Phase aus vernetzten Poly(acrylat)en aufweisen. Die Netzwerke auf Basis von Poly(acrylat) werden durch radikalische Polymerisation niedermolekularer Acrylate erhalten, denen als Vernetzer ein Dimethacrylat zugegeben wird. Die Monomere werden durch Quellung der Netzwerke aus Oligo[(rac-lactat)-co-glykolat]tetrolen und einem Diisocyanat in einer Lösung des Dimethacrylats und der flüssigen Acrylate aufgenommen. Die eingequollenen Acrylate können anschließend photochemisch polymerisiert werden. Vorteilhaft bei dieser Darstellungstechnik ist, dass definierte, bereits charakterisierte Netzwerke mit Oligo[(rac-lactat)-co-glykolat]segmenten eingesetzt werden. Mögliche Änderungen der thermischen oder mechanischen Eigenschaften können daher direkt auf die Bildung des Poly(acrylat)netzwerks zurückgeführt werden. Bei der Herstellung von sequenziellen IPNs durch Aufnahme von niedermolekularen Monomeren in das bereits gebildete Netzwerk können Monomere mit funktionellen Gruppen, wie z. B. Hydroxyfunktionen, eingesetzt werden

Diese Polymersysteme basieren auf Netzwerken, die durch Kupplung von Oligo[(rac-lactat)-co-glykolat]tetrolen, mit einem Gehalt von Glykolat wie oben definiert, bei einer zahlenmittleren Molmasse wie oben definiert, mit TMDI gebildet werden. Als Poly(acrylat)komponente sind Poly(ethylacrylat), Poly(butylacrylat) und Poly(hexylacrylat) bevorzugt, die aufgrund der niedrigen Glasübergangstemperaturen zum Aufbau einer kautschukelastischen Phase geeignet sind. So weist Poly(ethylacrylat) einen Wert für $T_g$ von -24 °C auf. Bei Poly(butylacrylat) und Poly(hexylacrylat) liegen die Glasübergangstemperaturen bei -55 °C bzw. -57 °C. Eine Hydrophilierung der Materialien kann durch Verwendung von (2-Hydroxyethyl)acrylat als Monomer realisiert werden. Poly(hydroxyethylacrylat) weist in trockenem Zustand eine Glasübergangstemperatur von 35 °C bis 58 °C auf. Als Vernetzer bei der radikalischen Polymerisation kann ein Oligo (propylenglykol)dimethacrylat (M-PPG-560) mit einer zahlenmittleren Molmasse nach Herstellerangaben von 560 g $mol^{-1}$ eingesetzt werden. Die Hydrophilie der Materialien hat eine hohe Relevanz für potenzielle Anwendungen biodegradierbarer Formgedächtnispolymere im medizinischen Bereich. Sie beeinflusst beispielsweise das Freisetzungsverhalten von Wirkstoffen aus Polymermatrices oder die Interaktion von Zellen zur Implantatoberfläche Bei Verwendung von (2-Hydroxyethyl)acrylat wird daher eine Steuerung der Hydrophilie und der Wasseraufnahme des IPNs durch den Gehalt des Poly(acrylat)s im Netzwerksystem angestrebt.

Der Anteil an Acrylat in den interpenetrierenden Netzwerken liegt bevorzugt im Bereich von 10 bis 80 Gew.-%, basierend auf der Gesamtzusammensetzung, stärker bevorzugt im Bereich von 15 bis 75 Gew.-% und insbesondere im Bereich von 20 bis 60 Gew.-%.

Die folgenden Beispiele illustrieren die vorliegende Erfindung näher.

Beispiele

Thermoplastische Materialien mit Polyestersegmenten aus Caprolacton wurden mit den Wirkstoffen Gentamicin bzw. in Enoxacin beladen, mit Wirkstoffmengen von 1 bis 20 Gew.-%. Anschließend wurde der Schmelzpunkt der Caprolactonsegmente bestimmt. Im Vergleich mit einem nicht mit Wirkstoff beladenen Matrixpolymer (Beladung erfolgte durch Lösungsvermischung und anschließendes Trocknen) wurde lediglich eine unbeachtliche Veränderung des Schmelzpunktes gefunden.

Die oben hergestellten Probenkörper wurden weiter im Hinblick auf mechanische Eigenschaften untersucht. Dabei wurde insbesondere der E-Modul bestimmt. Der E-Modul, so wurde dabei gefunden, steigt bei den beladenen Materialien gegenüber dem unbeladenen Material leicht an.

Weiterhin wurden Polyestermethacrylat-Netzwerke mit Polyestersegmenten aus Caprolacton und Glykolid mit Nitrofurantoin, Enoxacin und Ethacridinlactat beladen (1 bis 2 Gew.-%) (Quellverfahren) und der E-Modul wurde bestimmt. Im Vergleich mit den unbeladenen Materialien zeigt sich, dass eine Beladung mit Wirkstoffen den E-Modul nicht wesentlich verändert.

Weiterhin wurden Freisetzungsversuche mit bioabbaubaren Matrixpolymeren durchgeführt. Dabei wurden die oben genannten Polyestermethacrylat-Netzwerke mit 5 % Gentamicin bzw. Enoxazin beladen. Probenkörper mit einer Größe von 1 x 2 $cm^2$ wurden aufgeschnitten und in ein Freisetzungsmedium gegeben (4 ml Phosphatpuffer, pH 7). Die Filmstücke wurden dabei in Zentrifugenröhrchen so gestellt, dass sie von der Pufferlösung von allen Seiten umspült

werden. Die Zentrifugenröhrchen wurden im Schüttelwasserbad bei 37° C aufbewahrt und in bestimmten Zeitabständen wurde das Freisetzungsmedium komplett ausgetauscht, um die freigesetzte Menge an Wirkstoff zu bestimmen.

Diese Versuche wurden mit Netzwerken auf Basis von Paradioxanon und Polylactid-co-glykolid durchgeführt. Es ergab sich eine im wesentlichen lineare Freisetzungsrate der Wirkungstoffe, ohne Auftreten unerwünschter Burst-Effekte.

Dieser Versuch wurde auch mit dem Wirkstoff Gentamicin mit einem Thermoplast-Material auf Grundlage von Paradioxanon und Caprolacton durchgeführt. Hier zeigte sich, dass am Anfang ein sehr schneller Freisetzungseffekt (Burst-Effekt) erfolgte, wonach ein Plateau innerhalb von 2 Tagen erreicht wurde.

Dieses Freisetzungssystem wurde durch Eintauchbeschichtung mit dem Matrixpolymer modifiziert. Weiterhin wurde ein Laminatsystem hergestellt, umfassend einen wirkstoffhaltigen Film, der auf beiden Seiten mit einem Polymer abgedeckt wurde.

Diese Wirkstofffreisetzungssysteme zeigen ein anderes Freisetzungsverhalten. Hier erfolgt die Freisetzung, wie bereits oben für die Systeme auf Basis von Paradioxanon und Polylactid-co-glykolid beschrieben, im wesentlichen linear, über mehrere Wochen.

Die oben geschilderten Laminatsysteme können eine wirkstoffhaltige Filmschicht umfassen oder mehrere wirkstoffhaltige Filmschichten, jeweils umgeben von Filmen aus reinem Polymer.

Freisetzungsversuche wurden auch mit Netzwerken auf Grundlage von Polylactid-co-glykolid (Zahlenmittel des Molgewichtes der Segmente ungefähr 10.000, 15 Gew.-% Glykolidanteil) durchgeführt, die Ethacridinlactat enthielten. Auch hier ergab sich eine im wesentlichen lineare Freisetzung, die am Anfang etwas schneller ablief.

Beladung der thermoplastischen Materialien

Die Beladung erfolgt mittels Dispersion der Arzneistoffe in der Polymerlösung (in chlorierten Lösemitteln) und anschließendes Trocknen. Die getrocknete Mischung wird zwischen Teflonfolien unter Aufschmelzen zu Filmen gepresst.

Die verwendeten Arzneistoffe können sowohl hydrophil als auch lipophil sein. Als hydrophile Modellsubstanz dient Gentamicin, als lipophile Enoxacin. Es lassen sich Wirkstoffgehalte von bis zu 20 gew% erzielen.

Beladung von Netzwerken durch Quellung

Die Wirkstoffbeladung der Polymernetzwerke (die in Form von dünnen filmen vorliegen, Gewicht ca. 60 mg) erfolgt durch Quellung in einem 100-fachen Überschuss (V/m) an Wirkstofflösung über einen definierten Zeitraum. Im Allgemeinen beträgt die Quellungsdauer 24 h (eine Sättigung der Wirkstoffaufnahme wird jedoch schon nach etwa 1,5 h erreicht). Dabei wird von gesättigten Lösungen von Enoxacin in Chloroform bzw. in Ethylacetat, von Nitrofurantoin in Dioxan und von Ethacridinlactat in einem Lösungsmittelgemisch gleicher Massenanteile an Chloroform bzw. Ethylacetat und 2-Propanol ausgegangen. Anschließend werden die gequollenen Materialien aus der Lösung genommen. Die Polymernetzwerke werden bei 60 °C im Vakuum (1 mbar) getrocknet. In Tab. 1 sind die durch Quellung wirkstoffbeladenen Netzwerke in Abhängigkeit von der verwendeten gesättigten Wirkstofflösung aufgeführt.

Tab 1 : Beispiele zur Wirkstoffbeladung von Polymernetzwerken durch Quellung in einer gesättigten Wirkstofflösung.

| Beispiel | Netzwerk | Gesättigte Wirkstofflösung |
|---|---|---|
| 1 | N-CG(14)-3 | Enoxacin-Ethylacetat |
| 2 | N-CG(14)-3 | Ethacridinlactat-Ethylacetat/2-Propanol (1:1 w/w) |
| 3 | N-CG(14)-5 | Enoxacin-Ethylacetat |
| 4 | N-CG(14)-5 | Ethacridinlactat-Ethylacetat/2-Propanol (1:1 w/w) |
| 5 | N-CG(14)-7 | Enoxacin-Ethylacetat |
| 6 | N-CG(14)-7 | Ethacridinlactat-Ethylacetat/2-Propanol (1:1 w/w) |
| 7 | N-CG(14)-10 | Enoxacin-Ethylacetat |
| 8 | N-CG(14)-10 | Enoxacin-Chloroform |
| 9 | N-CG(14)-10 | Nitrofurantoin-Dioxan |
| 10 | N-CG(14)-10 | Ethacridinlactat-Ethylacetat/2-Propanol (1:1 w/w) |

(fortgesetzt)

| Beispiel | Netzwerk | Gesättigte Wirkstofflösung |
|---|---|---|
| 11 | N-CG(14)-10 | Ethacridinlactat-Chloroform/2-Propanol (1:1 w/w) |
| 12 | N-CG(0)-10 | Enoxacin-Ethylacetat |
| 13 | N-CG(0)-10 | Ethacridinlactat-Ethylacetat/2-Propanol (1:1 w/w) |
| 14 | N-CG(12)-10 | Enoxacin-Ethylacetat |
| 15 | N-CG(12)-10 | Ethacridinlactat-Ethylacetat/2-Propanol (1:1 w/w) |
| 16 | N-CG(21)-10 | Enoxacin-Ethylacetat |
| 17 | N-CG(21)-10 | Ethacridinlactat-Ethylacetat/2-Propanol (1:1 w/w) |
| 18 | N-CG(30)-10 | Enoxacin-Ethylacetat |
| 19 | N-CG(30)-10 | Ethacridinlactat-Ethylacetat/2-Propanol (1:1 w/w) |
| 20 | AB-CG(0)-10 | Enoxacin-Ethylacetat |
| 21 | AB-CG(0)-10 | Ethacridinlactat-Ethylacetat/2-Propanol (1:1 w/w) |
| 22 | AB-CG(12)-10 | Enoxacin-Ethylacetat |
| 23 | AB-CG(12)-10 | Ethacridinlactat-Ethylacetat/2-Propanol (1:1 w/w) |
| 24 | AB-CG(14)-10 | Enoxacin-Ethylacetat |
| 25 | AB-CG(14)-10 | Ethacridinlactat-Ethylacetat/2-Propanol (1:1 w/w) |
| 26 | AB-CG(21)-10 | Enoxacin-Ethylacetat |
| 27 | AB-CG(21)-10 | Ethacridinlactat-Ethylacetat/2-Propanol (1:1 w/w) |
| 28 | N-CG(14)TOH-5 | Enoxacin-Ethylacetat |
| 29 | N-CG(14)TOH-5 | Ethacridinlactat-Ethylacetat/2-Propanol (1:1 w/w) |
| 30 | N-LG(18)-10 | Enoxacin-Chloroform |
| 31 | N-LG(18)-10 | Nitrofurantoin-Dioxan |
| 32 | N-LG(18)-10 | Ethacridinlactat-Chloroform/2-Propanol (1:1 w/w) |

N-CG = Netzwerk aus Caprolacton-co-glycolid Segmenten
AB-CG-10 = AB Netzwerk aus Caprolacton-co-glycolid Segmenten, Copolymer n-Butylacrylat
N-CG(14)TOH-5 = Copolyesterurethannetzwerk aus Oligo[(ε-hydroxycaproat]-co-glycolat]tetraol und Diisocyanat
N-LG(18)-10 = Copolyesterurethannetzwerk aus Oligo[(rac-lactat)-co-glycolatlietraol und Diisocyanat
Die Zahlen in Klammern geben den den molaren Anteil an Glycolat an, bezogen auf das eingesetzte Makromonomer, die anderen Zahlen benennen Mn für die eingesetzten Makromonomere, jeweils in 1000 g/mol (5 = 5000 g/mol)

Tabelle 2 zeigt exemplarisch den Wirkstoffgehalt einiger Netzwerke - beladen durch das Quellverfahren - bestimmt durch verschiedene Methoden.

Tab. 2: Wirkstoffgehalt von Netzwerken. $\mu_{WS}$ ist der Wirkstoffanteil in der Matrix bezogen auf die Gesamtmasse, Q der Quellungsgrad in der Wirkstofflösung, $k_v$ der Verteilungskoeffizient und δ der Löslichkeitsparameter der Wirkstoffe.

| Netzwerk/ Wirkstoff | $\mu_{ws}$ (1) | $\mu_{ws}$ (2) | $\mu_{ws}$ (3) | Q | $k_v$ | δ |
|---|---|---|---|---|---|---|
| | Mass.-% | Mass.-% | Mass.% | Vol.-% | - | 1/2 $MPa^{1/2}$ |
| N-CG(14)-10 / Enoxacin$^+$ | 0,72 | | | 930 ± 60 | 0,33 | 29,1 |

(fortgesetzt)

| Netzwerk/ Wirkstoff | $\mu_{ws}$ (1) | $\mu_{ws}$ (2) | $\mu_{ws}$ (3) | Q | $k_v$ | $\delta$ |
|---|---|---|---|---|---|---|
| | Mass.-% | Mass.-% | Mass.% | Vol.-% | - | 1/2 $MPa^{1/2}$ |
| N-CG(14)-10/ Nitrofurantoin | 1,65 | 2,2 | 2,7 | 910 ± 80 | 0,63 | 31,7 |
| N-CG(14)-10 / Ethacridinlactat | 0,60 | 0,5 | 1,6 | 530 ± 10 | 0,47 | 28,6 |
| N-LG(18) / Enoxacin | 2,56 | | | 740 ± 20 | 2,35 | 28,08 |
| N-LG(18) / Nitrofurantoin | 1,40 | | | 790 ± 10 | 0,50 | 31,73 |
| N-LG(18) / Ethacridinlactat | 1,49 | | | 390 ± 10 | 1,43 | 27,62 |

(1) UV/VIS-spektroskopische Gehaltsbestimmung nach der Methanolyse des Netzwerks
(2) Gehaltsbestimmung durch Massenzunahme des Netzwerks nach Beladung
(3) Gehaltsbestimmung berechnet aus der Konzentrationsabnahme des Wirkstoffs in der Lösung

Beladung von Netzwerken in situ und Vernetzung

Zunächst wird eine 10%ige (m/V) Lösung der Präpolymere (Dimethacrylate) in einem Lösungsmittelgemisch gleicher Massenanteile an Dichlormethan und 2-Propanol angefertigt. Ein Anteil zwischen 0,2 Mass.-% und 6,6 Mass.-% Ethacridinlactat (bezogen auf die Gesamtmasse der wirkstoffhaltigen Matrix) wird zugefügt. Diese Lösung wird bei 50 °C eingeengt und anschließend bei 70 °C etwa 2 h im Vakuum (1 mbar) getrocknet. Ausgehend von dieser Zweikomponenten-Mischung wird die Vernetzung, wie folgt beschrieben, durchgeführt.

Die photochemische Vernetzung der Makrodimethacrylate erfolgt zwischen zwei Glasplatten unter Einsatz eines Heraeus Noble Light Excimer Laborsystems (308 nm). Die Glasform befindet sich in einem Abstand von 7,5 cm auf einer regelbaren Heizplatte bei 70 °C ± 2 °C unterhalb der UV-Röhren. Die Wärmeübertragung von der Heizplatte zu der Glasplatte wird durch einen Metallblock gewährleistet. Die Bestrahlungsdauer der Proben beträgt 30 min für die Netzwerke N-CG und 60 min für die AB-Netzwerke AB-CG.

Tab. 3: Bezeichnung, Zusammensetzung und Quellverhalten der Netzwerke mittels *in situ*-Inkorporierung von Ethacridinlactat. Molarer Glycolatanteil $\chi_G$ und zahlenmittlere Molmasse $M_n$ der verwendeten Makrodiethacrylate nach $^1$H-NMR-Spektroskopie; Massenanteil des Wirkstoffs $\mu_{ws}$ bezogen auf die Gesamtmasse. Q ist der Quellungsgrad in $CHCl_3$ und G der Gelgehalt

| Netzwerk | $\chi_G$ | $M_n$ | $\mu_{ws}$ | Q | G |
|---|---|---|---|---|---|
| | mol% | g $mol^{-1}$ | Mass.-% | Vol.% | Mass.-% |
| N-CG(14)-3-Etha(1)Dsp | 13 | 3500 | 1,0 | 610 ± 10 | 71 |
| N-CG(14)-5-Etha(1)Dsp | 14 | 4900 | 1,0 | 640 ± 40 | 92 |
| N-CG(14)-10-Etha(0,2)Dsp | 14 | 12800 | 0,2 | 900 ± 10 | 84 |
| N-CG(14)-10-Etha(1)Dsp | 14 | 12800 | 1,0 | 980 ± 20 | 85 |
| N-CG(14)-10-Etha(2)Dsp | 14 | 12800 | 2,0 | 1020 ± 110 | 62 |
| N-CG(14)-10-Etha(4)Dsp | 14 | 12800 | 3,9 | 980 ± 40 | 75 |
| N-CG(14)-10-Etha(5)Dsp | 14 | 12800 | 4,8 | 1040 ± 20 | 71 |

(fortgesetzt)

| Netzwerk | $\chi_G$ | $M_n$ | $\mu_{ws}$ | Q | G |
|---|---|---|---|---|---|
| | mol% | g $mol^{-1}$ | Mass.-% | Vol.% | Mass.-% |
| N-CG(14)-10-Etha(6)Dsp | 14 | 12800 | 5,7 | 1080 ± 150 | 53 |
| N-CG(0)-10-Etha(1)Dsp | 0 | 10800 | 1,0 | 850 ± 50 | 93 |
| N-CG(21)-10-Etha(1)Dsp | 21 | 13500 | 1,0 | 1040 ± 20 | 67 |

Hydrolytische Abbauexperimente

Die Hydrolyseexperimente werden an planaren Probekörpern mit einer Fläche von 10 mm × 15 mm und einer Dicke von etwa 0,2 mm (Poly(esterurethan)netzwerke) bzw. 0,5 mm (photochemisch vernetzte Polymere) in 15 mL Zentrifugengefäßen aus Polypropylen durchgeführt. Die Probekörper werden vor dem Experiment jeweils dreimal mit Hexan-Fraktion gewaschen und im Vakuum (1 mbar) getrocknet. Anschließend wird die Masse ($m_{ini}$) jeder Probe bestimmt. Als Abbaumedium dient eine phosphatgepufferte wässrige Lösung aus $Na_2HPO_4$ (0,1 mol$\mu$L$^{-1}$) und $KH_2PO_4$ (0,063 mol$\mu$L$^{-1}$) mit pH 7,0. Die Kapazität der Pufferlösung eines Volumens von 15 mL reicht aus, um 85 mmol Säure abzupuffern. Um das Wachstum von Mikroorganismen während des Abbaus zu vermeiden, wird der Pufferlösung 0,25 g□L$^{-1}$ Natriumazid zugesetzt. Das Hydrolyseexperiment wird ohne Austausch des Abbaumediums unter regelmäßiger Kontrolle des pH-Werts in einem temperierbaren Schüttelwasserbad bei 37 °C oder 70 °C mit 60 Rotationen pro Minute durchgeführt. Die Temperatur wird dabei auf ± 0,1 K genau geregelt.

Die Anzahl der separat abgebauten Proben einer Reihe entspricht der Zahl der festgesetzten Messzeitpunkte. Zu dem jeweilig definierten Zeitpunkt wird die Probe dem Hydrolysemedium entnommen. Nach dem Abtupfen mit Zellstoff wird ihre Masse ($m_h$) bestimmt. Anschließend wird die Probe bei 30 °C im Vakuum (1 mbar) getrocknet und wiederum gewogen ($m_{ht}$). Anhand dieser Messgrößen wird das Massenverhältnis $\mu_{rel}$ und die Wasseraufnahme H in Mass.-% während des hydrolytischen Abbaus bestimmt.

$$(1) \qquad \mu_{rel} = \frac{m_{ht}}{m_{ini}}$$

$$(2) \qquad H = \frac{m_h - m_{ini}}{m_{ini}}$$

Freisetzungsexperimente der wirkstoffhaltigen Polymernetzwerke

Die Bestimmung der Wirkstofffreisetzung aus Polymernetzwerken erfolgt anhand von planaren Probekörpern einer Größe von 1 cm x 1 cm mit einer Dicke von etwa 0,2 mm (Poly-(esterurethan)netzwerke) bzw. 0,5 mm (photochemisch vernetzte Polymere). Die Probekörper werden in verschließbaren 15 mL Polypropylen-Zentrifugenröhrchen von allen Seiten von 4 mL des Freisetzungsmediums umspült. Als Freisetzungsmedium dient eine phosphatgepufferte wässrige Lösung aus $Na_2HPO_4$ (0,1 mol$\mu$L$^{-1}$) und $KH_2PO_4$ (0,063 mol$\mu$L$^{-1}$) mit pH 7,0. Das Freisetzungsexperiment wird in einem temperierbaren Schüttelwasserbad bei 37 °C mit 60 Rotationen pro Minute durchgeführt. Die Temperatur wird dabei auf ± 0,1 K genau geregelt. Durch den vollständigen Austausch des Freisetzungsmediums in bestimmten Zeitabständen wird ermöglicht, dass eine Wirkstoffkonzentration von 10% der Sättigungskonzentration im Freisetzungsmedium nicht überschritten wird (*sink* Bedingungen). Der Versuch ist beendet, wenn der gesamte Wirkstoff freigesetzt ist. Die Erstellung des Freisetzungs-Profils erfolgt durch die Bestimmung des freigesetzten Wirkstoffs mittels UV-Vis-Spektrometrie. Die resultierenden Freisetzungsprofile repräsentieren das arithmetische Mittel aus der parallelen Bestimmung der Freisetzung von jeweils drei Proben eines Materials.

Falls das Freisetzungsexperiment beendet wird, bevor der Wirkstoff vollständig aus der Matrix freigesetzt ist, ist eine Gehaltsbestimmung des verbleibenden Wirkstoffs in dem Polymernetzwerk erforderlich. Die Matrix wird dem Freisetzungsmedium entnommen, mit Zellstoff abgetupft und bei 35 °C im Vakuum (1 mbar) getrocknet. Unter Zugabe

von 5 mL Chloroform werden die Netzwerke gequollen und mit Hilfe eines Glasstabs mechanisch zerstört. Unter Zugabe von 5 mL Chloroform werden die gequollenen, enoxacinhaltigen Fragmente extrahiert. Bei ethacridinlactathaltigen Netzwerken erfolgt die Extraktion unter Zugabe von 5 mL 2-Propanol. Nach 2 h wird über eine Glasfritte P4 filtriert und die Wirkstoffkonzentration des Filtrat photometrisch bestimmt. Der in der Matrix verbliebene Wirkstoffanteil wird zu dem im Freisetzungsexperiment additiv ermittelten Anteil addiert.

**Patentansprüche**

1. Wirkstofffreisetzungssystem zur Steuerung der Wirkstofffreisetzung on-demand umfassend ein Form-Gedächtnis-Polymer (SMP-Material) und mindestens einen Wirkstoff, wobei der Form-Gedächtnis-Effekt zur Veräderung der Wirkstofffreisetzungsrate eingesetzt wird.

2. Wirkstofffreisetzungssystem nach Anspruch 1, wobei das SMP-Material eine oder mehrere Formen im Gedächtnis hat.

3. Wirkstofffreisetzungssystem nach einem der vorstehenden Ansprüche, wobei das SMP-Material biostabil oder bioabbaubar ist.

4. Wirkstofffreisetzungssystem nach einem der vorstehenden Ansprüche, wobei der Form-Gedächtnis-Effekt durch eine Temperaturveränderung, Licht oder eine Kombination davon ausgelöst wird.

5. Wirkstofffreisetzungssystem nach einem der vorstehenden Ansprüche, wobei das Wirkstofffreisetzungssystem ein Matrixsystem ist, wobei der mindestens eine Wirkstoff in der Matrix verteilt vorliegt.

6. Wirkstofffreisetzungssystem nach Anspruch 5, wobei das Wirkstofffreisetzungssystem eine Veränderung der Wirkstofffreisetzungsrate nach Auslösen des Form-Gedächtnis-Effekts zeigt.

7. Wirkstofffreisetzungssystem nach einem der Ansprüche 5 oder 6, wobei das SMP-Material Segmente umfasst, abgeleitet von Caprolacton, Lactid, Glykolid und Dioxanon.

8. Wirkstofffreisetzungssystem nach einem der Ansprüche 5 bis 7, wobei das Wirkstofffreisetzungssystem eine Beschichtung aufweist, zur Modifikation des Freisetzungsverhaltens und/oder der Gewebeverträglichkeit.

9. Wirkstofffreisetzungssystem nach Anspruch 5, wobei das Wirkstofffreisetzungssystem in Laminatform vorliegt, umfassend mindestens einen mit Wirkstoff beladenen Film aus einem SMP-Material, wobei dieser Film auf beiden Oberflächen mit einem an Wirkstoff freien Film laminiert ist.

10. Wirkstofffreisetzungssystem nach einem der Ansprüche 1 bis 3, wobei das Wirkstofffreisetzungssystem ein Reservoir an Wirkstoff umfasst und eine Beschichtung und/oder Membran aus einem SMP-Material.

11. Wirkstofffreisetzungssystem nach Anspruch 10, wobei das SMP-Material nach Auslösen des Form-Gedächtnis-Effekts die Freisetzungsrate des Wirkstoffs reguliert.

12. Wirkstofffreisetzungssystem nach einem der vorstehenden Ansprüche 1 bis 3, wobei das Wirkstofffreisetzungssystem ein Reservoir aus einem SMP-Material für den Wirkstoff umfasst und eine Beschichtung und/oder Membran.

13. Wirkstofffreisetzungssystem nach Anspruch 12, wobei der Form-Gedächtnis-Effekt zu einer Formänderung des Reservoirs eingesetzt wird, wodurch die Beschichtung und/oder Membran ihre Durchlässigkeit für den Wirkstoff ändert.

14. Wirkstofffreisetzungssystem nach einem der vorstehenden Ansprüche, wobei der Wirkstoff ein niedermolekularer oder hochmolekularer, hydrophiler oder hydrophober Wirkstoff ist.

15. Wirkstofffreisetzungssystem nach einem der vorstehenden Ansprüche, wobei das Wirkstofffreisetzungssystem als Beschichtung auf einem Implantat vorgesehen ist.

16. Wirkstofffreisetzungssystem nach einem der vorstehenden Ansprüche, wobei das Wirkstofffreisetzungssystem in

der Form von Nanopartikeln, Mikropartikeln, Filmen, Fäden, Zusammensetzungen für die transdermale Wirkstoffverabreichung oder Ähnlichem vorliegt.

**17.** Verfahren für die Herstellung eines Wirkstofffreisetzungssystems nach einem der vorstehenden Ansprüche, umfassend das Lösen eines Wirkstoffs in einem geeigneten Lösungsmittel, Einbringen eines Form-Gedächtnis-Netzwerks in die Lösung und Quellen des Netzwerks in der Gegenwart der Wirkstofflösung und Entfernen des gequollenen Netzwerks aus der Lösung.

**18.** Verfahren für die Herstellung eines Wirkstofffreisetzungssystems nach einem der vorstehenden Ansprüche, umfassend die Vernetzung von Präpolymeren in der Gegenwart eines Wirkstoffs.

**19.** Verfahren nach Anspruch 18, wobei der Wirkstoff in der zu vernetzenden Mischung gelöst oder dispergiert vorliegt.

**20.** Verwendung eines Wirkstofffreisetzungssystems nach einem der Ansprüche 1 bis 16 zur Herstellung eines Medikaments mit einer durch den Form-Gedächtnis-Effekt steuerbaren Wirkstofffreisetzung.

## Claims

**1.** Drug release system for controlling on-demand drug release, comprising a shape memory polymer (SMP material) and at least one drug, wherein the shape memory effect is used to vary the rate of drug release.

**2.** Drug release system according to Claim 1, wherein the SMP material has one or more shapes in its memory.

**3.** Drug release system according to either of the preceding claims, wherein the SMP material is biostable or biodegradable.

**4.** Drug release system according to any of the preceding claims, wherein the shape memory effect is triggered by a temperature change, light or a combination thereof.

**5.** Drug release system according to any of the preceding claims, wherein the drug release system is a matrix system, wherein at least one drug is present distributed in the matrix.

**6.** Drug release system according to Claim 5, wherein the drug release system shows a change in the rate of drug release after triggering of the shape memory effect.

**7.** Drug release system according to either Claim 5 or 6, wherein the SMP material comprises segments derived from caprolactone, lactide, glycolide and dioxanone.

**8.** Drug release system according to any of Claims 5 to 7, wherein the drug release system has a coating, to modify release behaviour and/or tissue compatibility.

**9.** Drug release system according to Claim 5, wherein the drug release system is present in the form of a laminate comprising at least one drug-laden film made of an SMP material, wherein the film is laminated on both surfaces with a drug-free film.

**10.** Drug release system according to any of Claims 1 to 3, wherein the drug release system comprises a drug reservoir, and a coating and/or membrane made of an SMP material.

**11.** Drug release system according to Claim 10, wherein the SMP material regulates the rate of drug release after triggering of the shape memory effect.

**12.** Drug release system according to any of preceding Claims 1 to 3, wherein the drug release system comprises a reservoir made of an SMP material for the drug, and a coating and/or membrane.

**13.** Drug release system according to Claim 12, wherein the shape memory effect is used to change the shape of the reservoir, causing the coating and/or membrane to change their/its permeability to the drug.

14. Drug release system according to any of the preceding claims, wherein the drug is a low-molecular-weight or high-molecular-weight, hydrophilic or hydrophobic drug.

15. Drug release system according to any of the preceding claims, wherein the drug release system is intended as a coating on an implant.

16. Drug release system according to any of the preceding claims, wherein the drug release system is present in the form of nanoparticles, microparticles, films, threads, compositions for transdermal drug administration or similar.

17. Method for the manufacture of a drug release system according to any of the preceding claims, comprising dissolving a drug in a suitable solvent, introducing a shape memory network into the solvent and swelling the network in the presence of the drug solution and removing the swollen network from the solution.

18. Method for the manufacture of a drug release system according to any of the preceding claims, comprising crosslinking prepolymers in the presence of a drug.

19. Method according to Claim 18, wherein the drug is present dissolved or dispersed in the mixture to be crosslinked.

20. Use of a drug release system according to any of Claims 1 to 16 in the manufacture of a medicament providing drug release controllable by the shape memory effect.

**Revendications**

1. Système de libération de substance active pour le contrôle de la libération de substance active "sur demande", comprenant un polymère à mémoire de forme (matériau SMP) et au moins une substance active, où l'effet de mémoire de forme est utilisé pour la modification de la vitesse de libération de la substance active.

2. Système de libération de substance active selon la revendication 1, où le matériau SMP a une ou plusieurs formes en mémoire.

3. Système de libération de substance active selon l'une quelconque des revendications précédentes, où le matériau SMP est biostable ou biodégradable.

4. Système de libération de substance active selon l'une quelconque des revendications précédentes, où l'effet de mémoire de forme est déclenché par une modification de température, la lumière ou une combinaison de celles-ci.

5. Système de libération de substance active selon l'une quelconque des revendications précédentes, où le système de libération de substance active est un système de matrice, ladite au moins une substance active se trouvant sous forme répartie dans la matrice.

6. Système de libération de substance active selon la revendication 5, où le système de libération de substance active présente une modification de la vitesse de libération de la substance active après déclenchement de l'effet de mémoire de forme.

7. Système de libération de substance active selon l'une quelconque des revendications 5 ou 6, où le matériau SMP comprend des segments dérivés de caprolactone, de lactide, de glycolide et de dioxanone.

8. Système de libération de substance active selon l'une quelconque des revendications 5 à 7, où le système de libération de substance active présente un revêtement pour la modification du comportement de libération et/ou de la compatibilité avec les tissus.

9. Système de libération de substance active selon la revendication 5, où le système de libération de substance active se trouve sous forme de laminé, comprenant au moins un film chargé de substance active en un matériau SMP, ce film étant laminé sur les deux surfaces avec un film exempt de substance active.

10. Système de libération de substance active selon l'une quelconque des revendications 1 à 3, où le système de libération de substance active comprend un réservoir de substance active et un revêtement et/ou une membrane

en matériau SMP.

**11.** Système de libération de substance active selon la revendication 10, où le matériau SMP régule, après déclenchement de l'effet de mémoire de forme, la vitesse de libération de la substance active.

**12.** Système de libération de substance active selon l'une quelconque des revendications précédentes 1 à 3, où le système de libération de substance active comprend un réservoir en matériau SMP pour la substance active et un revêtement et/ou une membrane.

**13.** Système de libération de substance active selon la revendication 12, où l'effet de mémoire de forme est utilisé pour une modification de forme du réservoir, suite à quoi le revêtement et/ou la membrane modifie(ent) sa(leur) perméabilité pour la substance active.

**14.** Système de libération de substance active selon l'une quelconque des revendications précédentes, où la substance active est une substance active de bas ou de haut poids moléculaire, hydrophile ou hydrophobe.

**15.** Système de libération de substance active selon l'une quelconque des revendications précédentes, où le système de libération de substance active est prévu sous forme de revêtement sur un implant.

**16.** Système de libération de substance active selon l'une quelconque des revendications précédentes, où le système de libération de substance active se trouve sous forme de nanoparticules, de microparticules, de films, de fils, de compositions pour l'administration transdermique de substance active ou analogue.

**17.** Procédé pour la fabrication d'un système de libération de substance active selon l'une quelconque des revendications précédentes, comprenant la dissolution d'une substance active dans un solvant approprié, l'introduction d'un réseau à mémoire de forme dans la solution et gonflement du réseau en présence de la solution de substance active et élimination du réseau gonflé de la solution.

**18.** Procédé pour la fabrication d'un système de libération de substance active selon l'une quelconque des revendications précédentes, comprenant la réticulation de prépolymères en présence d'une substance active.

**19.** Procédé selon la revendication 18, où la substance active se trouve sous forme dissoute ou dispersée dans le mélange à réticuler.

**20.** Utilisation d'un système de libération de substance active selon l'une quelconque des revendications 1 à 16 pour la préparation d'un médicament avec une libération de substance active contrôlable par l'effet de mémoire de forme.

**Figuren**

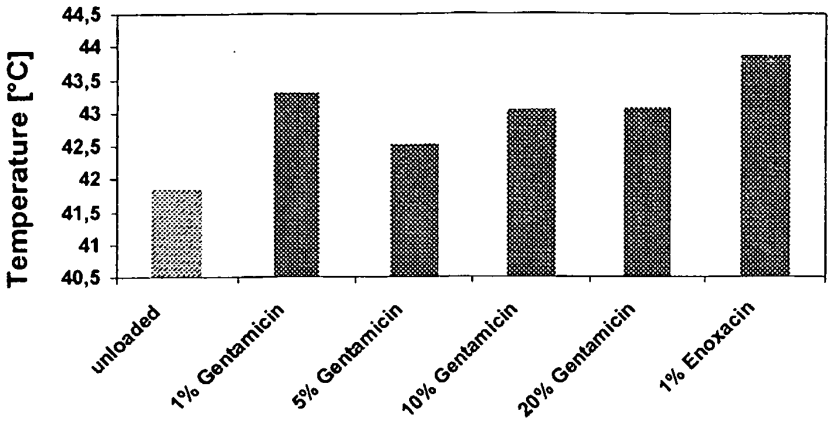


Fig 1: Schmelztemperatur eines thermoplast. Multiblockcopolymers aus Paradioxanon / Caprolacton beladen mit Wirkstoffen

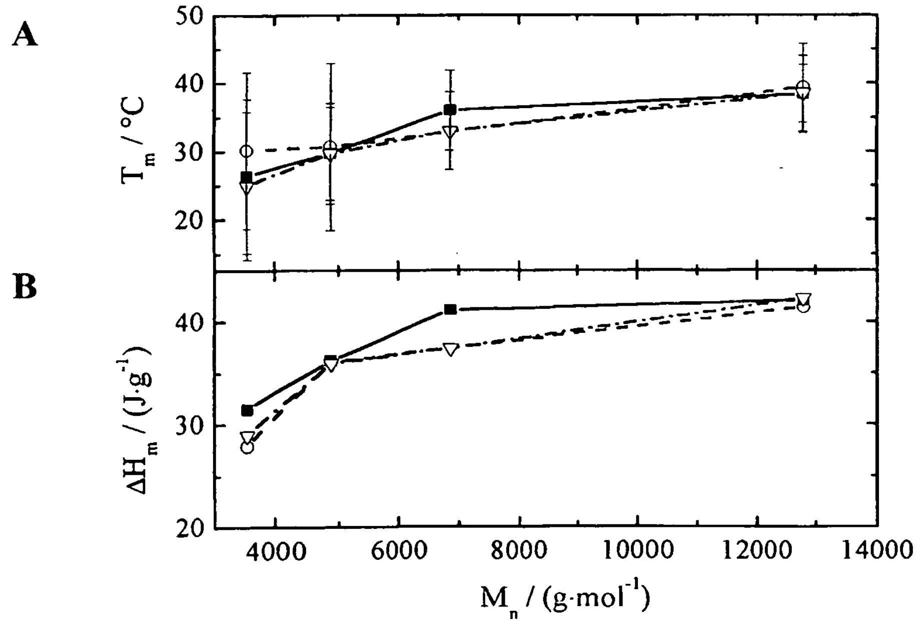


Fig 2 Thermische Eigenschaften von N-CG Netzwerken unterschiedlicher Segmentlängen mit und ohne Ethacridinlactatbeladung. Der Balken gibt die Breite des thermischen Übergangs an.

■ N-CG(14);

▼ N-CG(14)-Ethacridin; Wirkstoffbeladung durch Quellung
○ N-CG(14)-Etha(1)Dsp ; Wirkstoffbeladung durch Dispersion mit Präpolymer

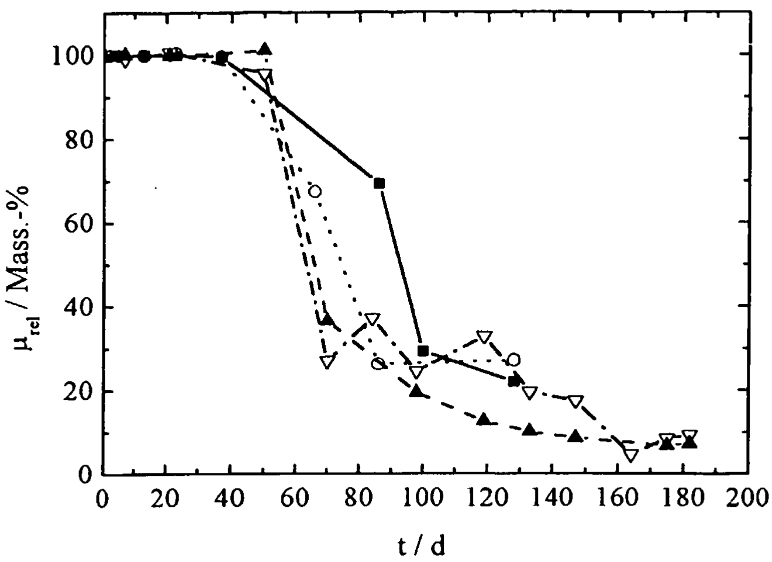


Fig 3: Relativer Massenverlust der wirkstoffhaltigen amorphen Netzwerke N-LG(18)-10 bei 37 °C in einer Phosphatpufferlösung (pH 7,0).
■ N-LG(18)-10
▼ N-LG(18)-10-Enoxacin
▲ N-LG(18)-10-Nitrofurantoin
○ N-LG(18)-10-Ethacridin

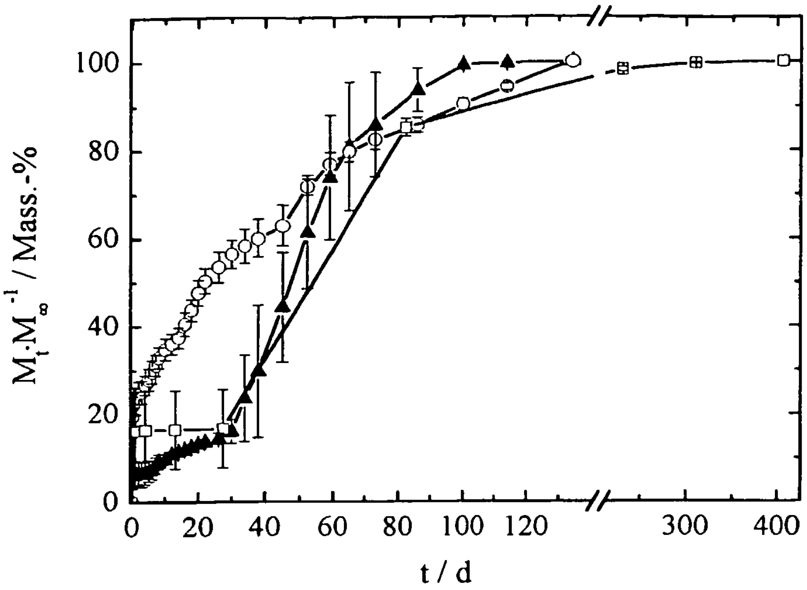


Fig 4: Wirkstofffreisetzung aus amorphen Netzwerken N-LG(18)-10 bei 37 °C in einer Phosphatpufferlösung (pH 7,0). Die Oberfläche, über die der Wirkstoff freigesetzt wird, beträgt 2 $cm^2$ und die Dicke der Matrix 0,2 mm.
$M_t * M_\infty^{-1}$ Massenanteil der aus dem Netzwerk freigesetzten Wirkstoffe
Ethacridinlactat (○)
Nitrofurantoin (▲)
Enoxacin (□)

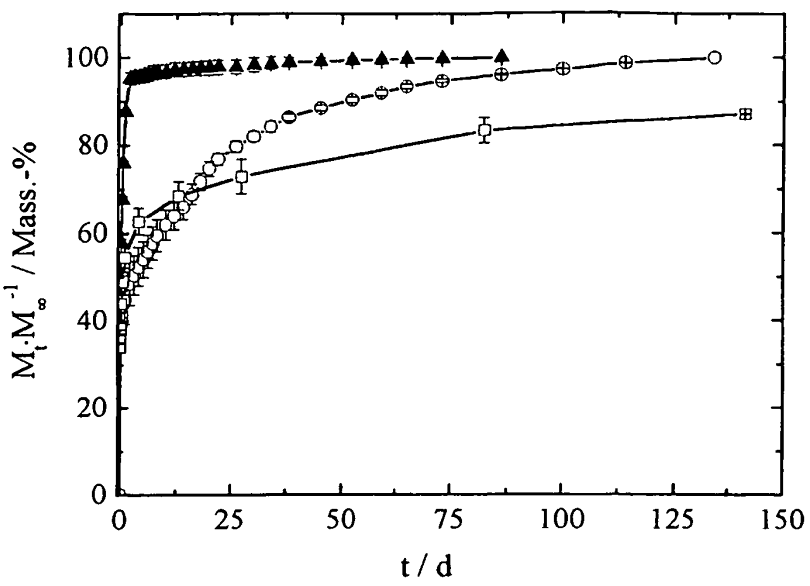


Fig 5: Wirkstofffreisetzung aus kristallinen Netzwerken N-CG(14)-10 bei 37 °C in einer Phosphatpufferlösung (pH 7,0). Die Oberfläche, über die der Wirkstoff freigesetzt wird, beträgt 2 $cm^2$ und die Dicke der Matrix 0,45 mm.
Massenanteil $M_t*M_\infty^{-1}$ der aus dem Netzwerk freigesetzten Wirkstoffe
Ethacridinlactat (○)
Nitrofurantoin (▲)
Enoxacin (□)

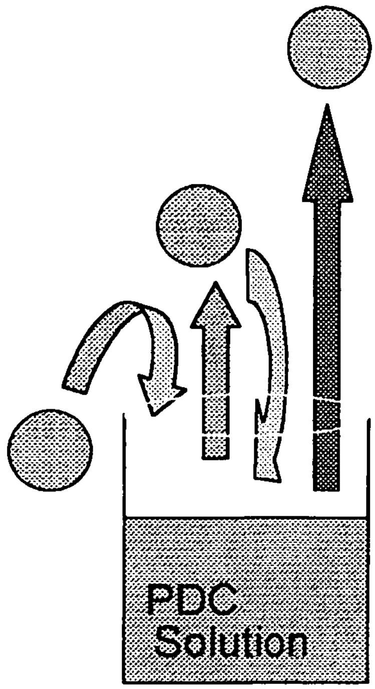


Fig 6: Methode des Dip-Coatings zur Modifizierung von Wirkstofffreisetzungssystemen

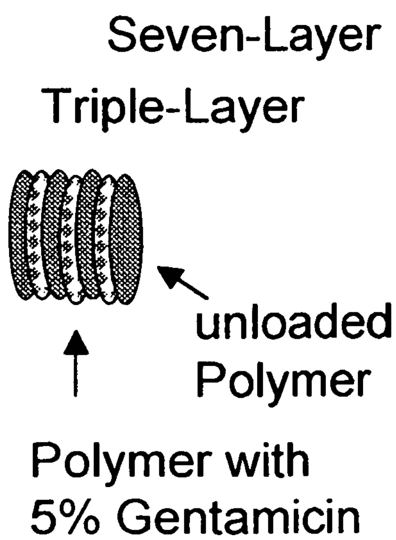


Fig 7: Aufbau von Layer-Systemen

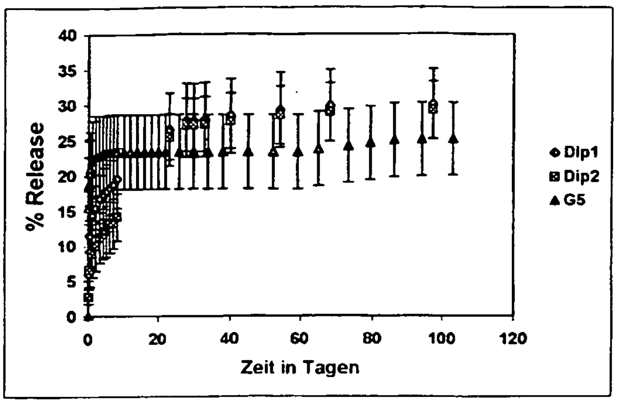


Fig 8: Modifizierung des Release von Gentamicin aus durch Dip Coating (G5 =5 wt.% Gentamicin enthalten) aus Paradioxanon/ Caprolacton Multiblockcopolymer.
Dip 1 einmal in Polymerlösung getaucht
Dip 2 zweimal in Polymerlösung getaucht

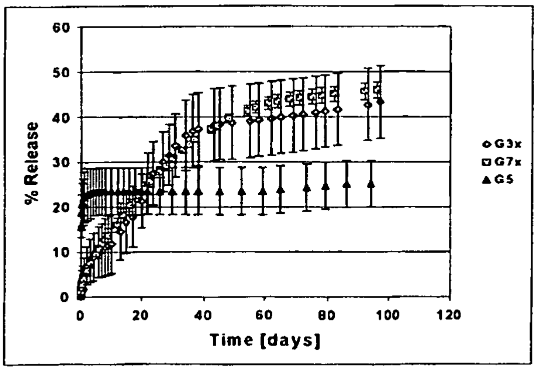


Fig 9: Modifizierung des Release von Gentamicin aus Paradioxanon/ Caprolacton Multiblockcopolymer durch Herstellung von Layer-Systemen (G5 = 5 wt.% Gentamicin enthalten)
G3x 3-Layer mit 5 wt.% Gentamicin im mittleren Film
G7x 7-Layer mit 3mal 5 wt.% Gentamicin-Filmen umgeben von reinem Poly merfilm

Fig 10

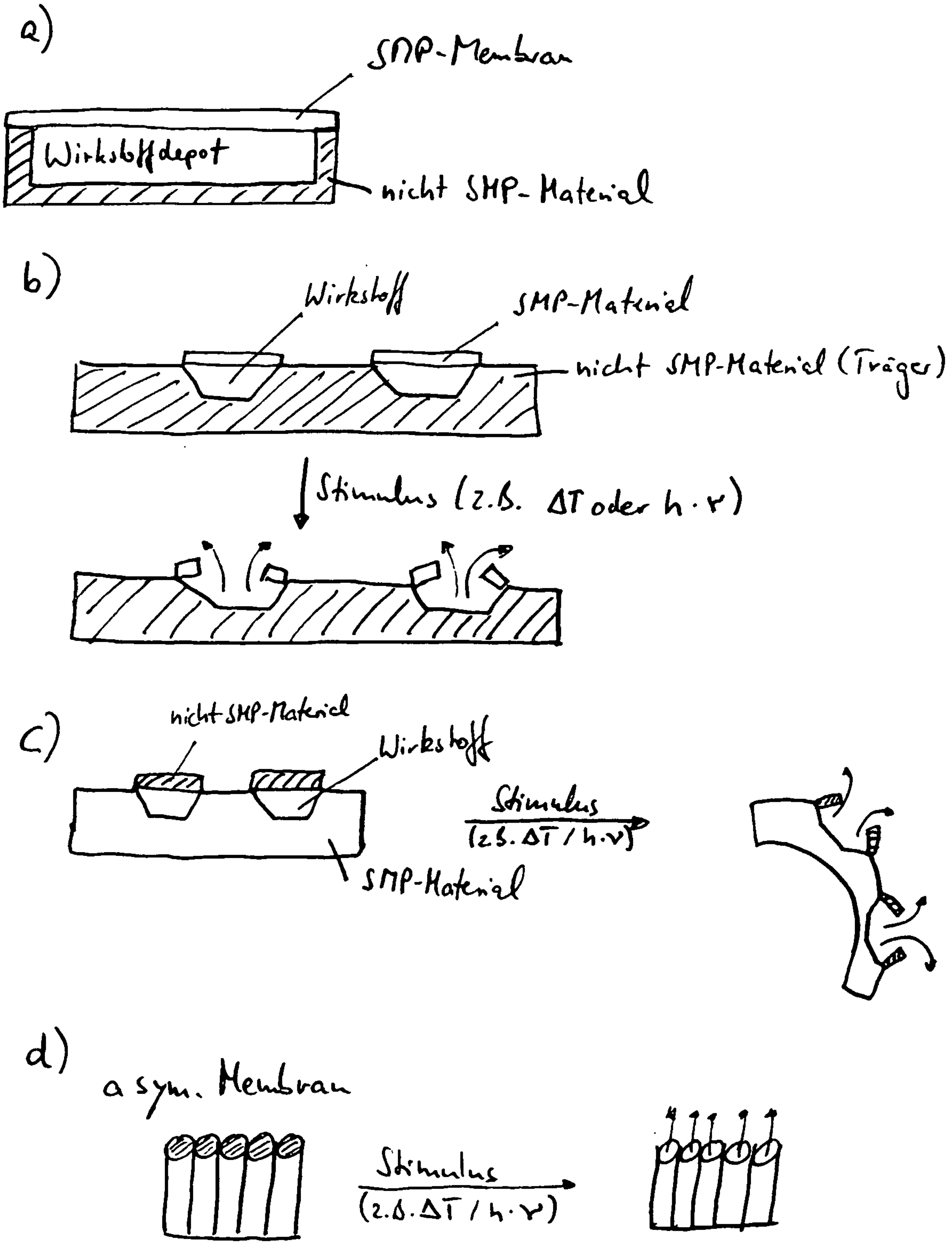
a)
SMP-Membran
Wirkstoffdepot
nicht SMP-Material
b)
Wirkstoff
SMP-Material
nicht SMP-Material (Träger)
Stimulus (z.B. ΔT oder h · ν)
c)
nicht SMP-Material
Wirkstoff
Stimulus
(z.B. ΔT / h·ν)
SMP-Material
d)
asym. Membran
Stimulus
(z.B. ΔT / h·ν)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0241929 A
- US 6160084 A
- WO 9942528 A
- WO 9942147 A
- EP 0415671 A
- EP 1000958 A
- EP 0422693 A
- US 200109982 B
- WO 03068288 A
- WO 9962576 A